# EUROPEAN PATENT APPLICATION

(11) **EP 1 502 919 A1**
(43) Date of publication of application: **02.02.2005**
(21) Application number: 03017076.5
(22) Date of filing: 28.07.2003
(51) Int. Cl.: C07K 14/435, G06F 19/00, G01N 33/68

(54) **Method for obtaining agents against insects by using the structural coordinates of ZAD grau**

(71) Applicant: Max-Planck-Gesellschaft zur Förderung der Wissenschaften e.V., 80539 München (DE)
(72) Inventor: Chung, Ho-Ryun, 37083 Göttingen (DE); Wahl, Markus, 37077 Göttingen (DE); Jauch, Ralf, 37075 Göttingen (DE)
(74) Representative: Epping Hermann & Fischer

(57) **Abstract**

The present invention discloses a method for obtaining agents interacting with a ZAD_{Grau} molecule by using the structural coordinates obtained from crystals of ZAD_{Grau}. These agents can serve as insecticides.

## Description

The present invention relates to methods for obtaining agents against insects by using the structural coordinates of a zinc finger-associated domain (ZAD_{Grau}) of the transcription factor Grauzone (Grau). This invention is also related to crystals comprising ZAD_{Grau} molecules and to methods of obtaining 3-dimensional models of ZAD_{Grau}-like molecules of unknown structure by using the structural coordinates of ZAD_{Grau}.

Resistance to antiparasitic compounds among insects is a wide-spread problem. A large group of insecticides, the organophosphates and the carbamates inactivate the insecticidal acetylcholinesterase, thereby terminating synaptic transmission through hydrolysis of the neurotransmitter acetylcholine, leading to paralysis and death of the insects. Insensitivity of the insects towards the organophosphates and carbamates was shown to be conferred by a single amino-acid substitution in the acetylcholinesterase (Weill et al., 2003). Therefore there is a continuos need to reveal new targets for the development of new insecticides. These new targets are preferably proteins, performing vital functions for the insects.

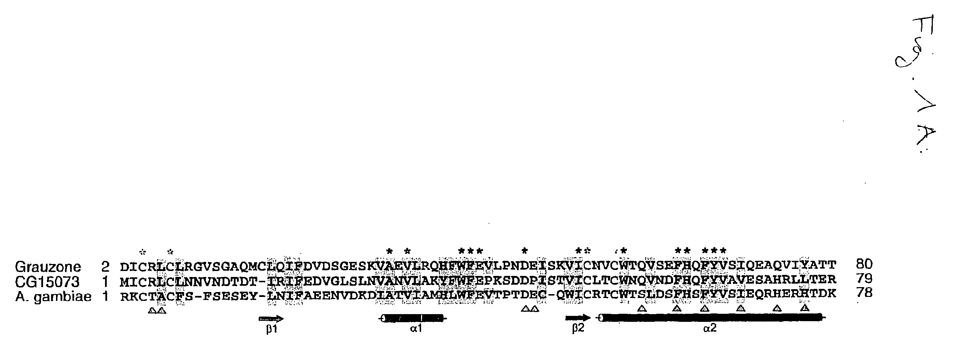

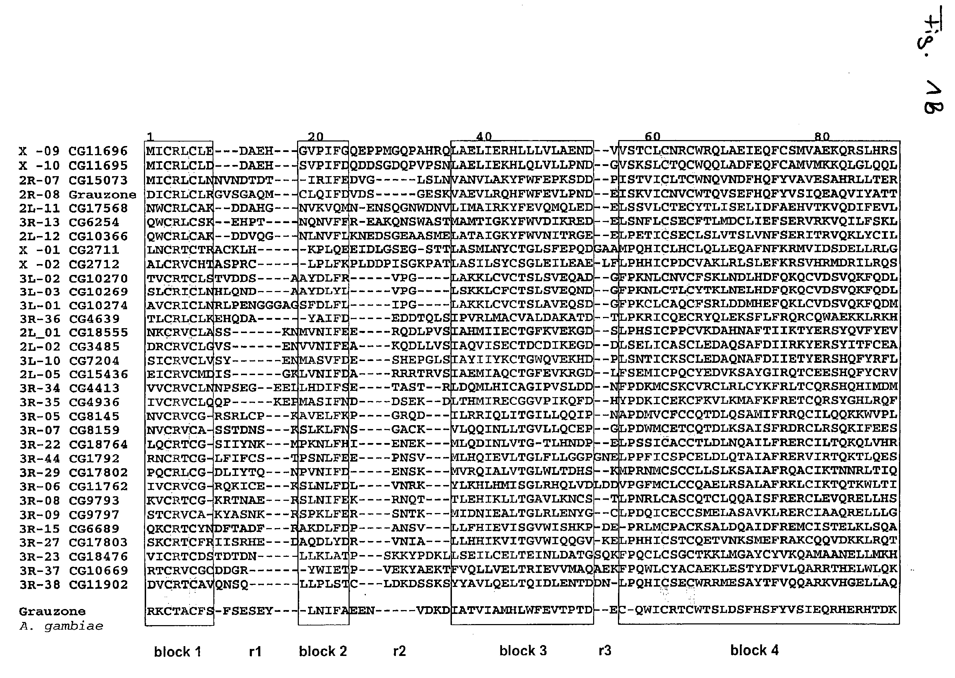

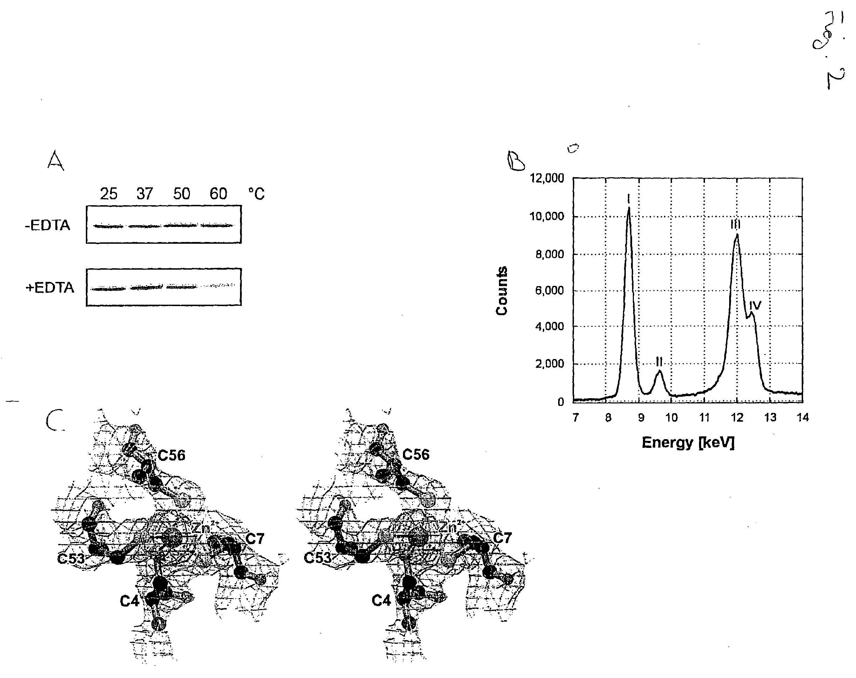

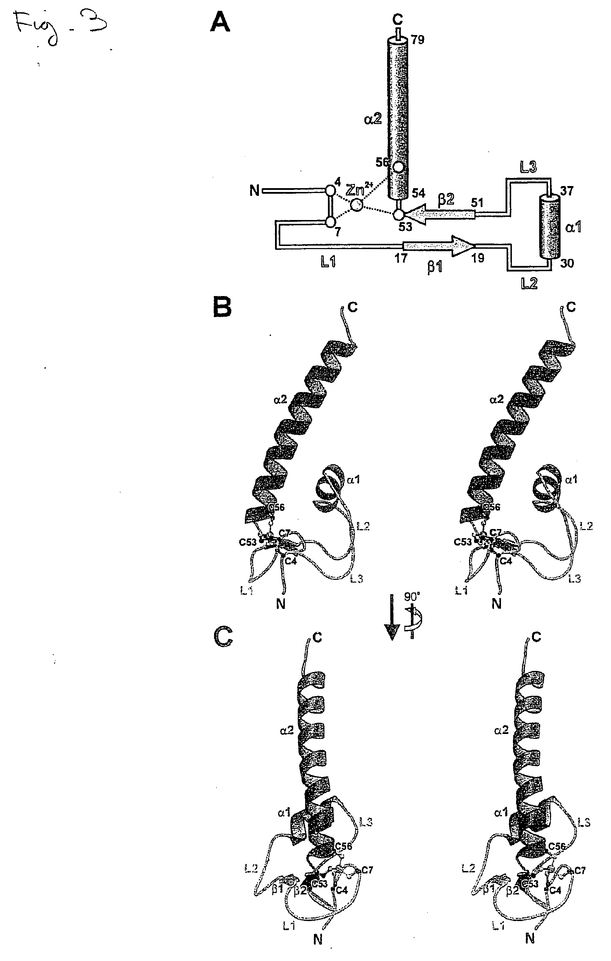

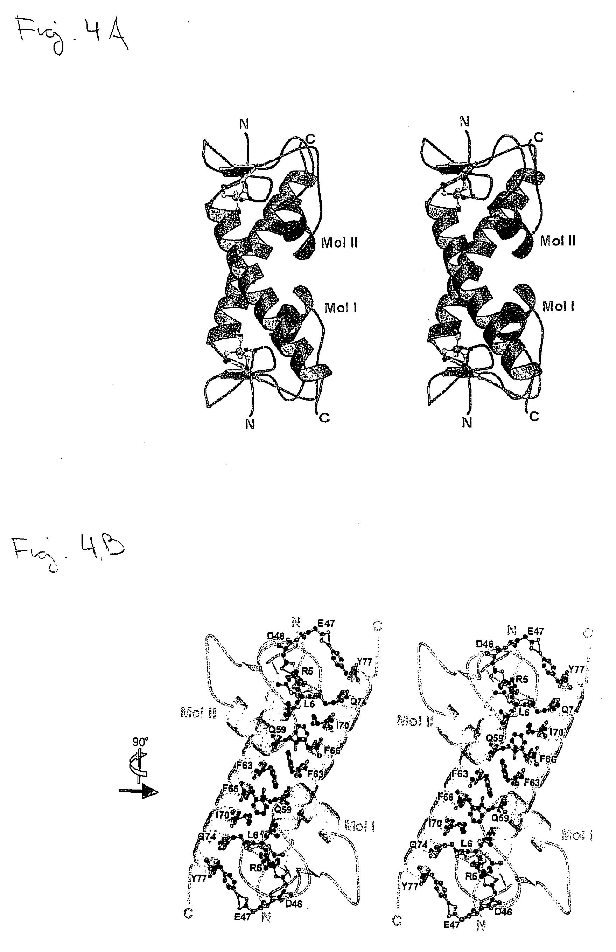

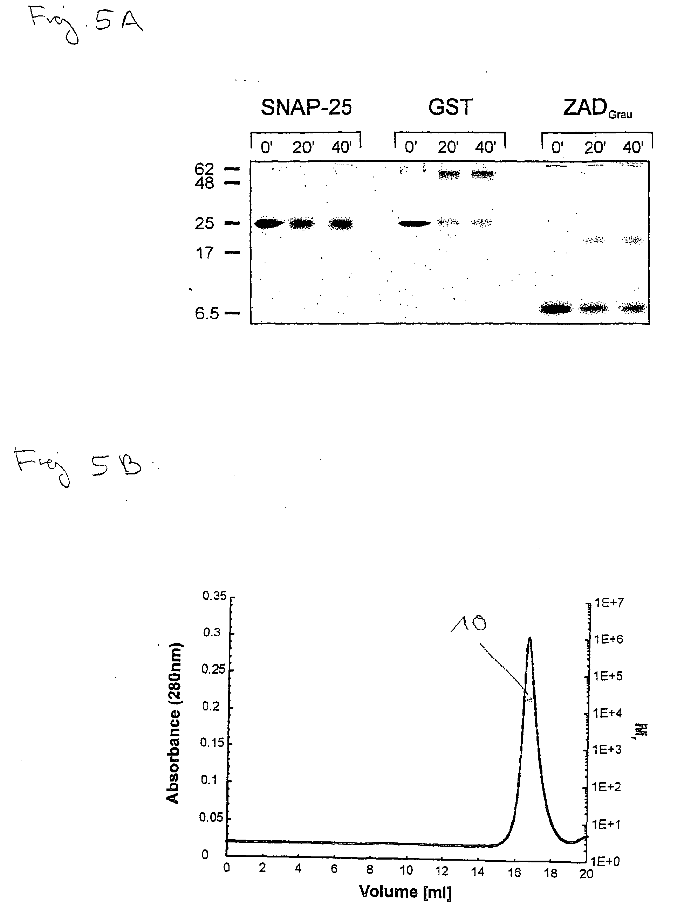

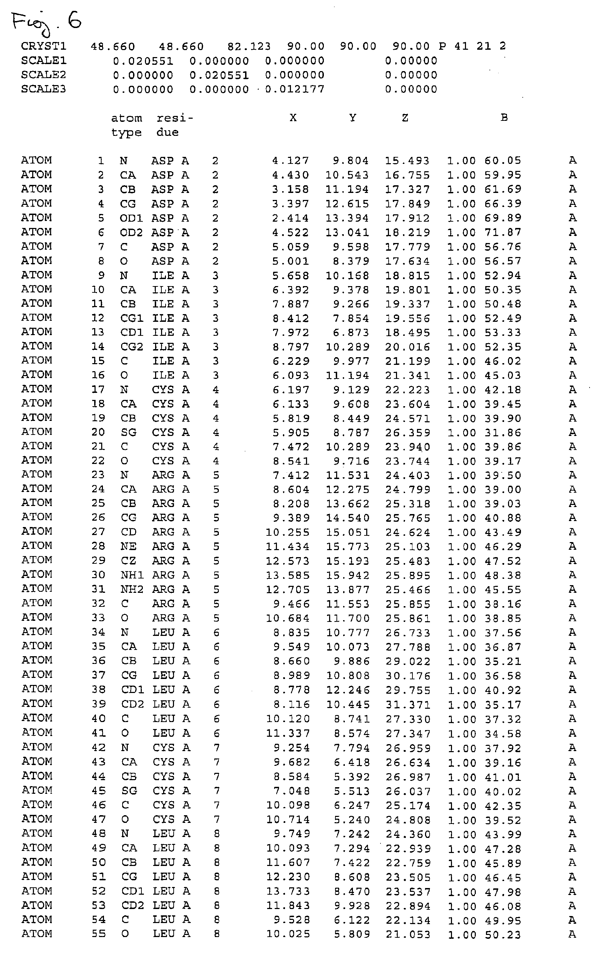

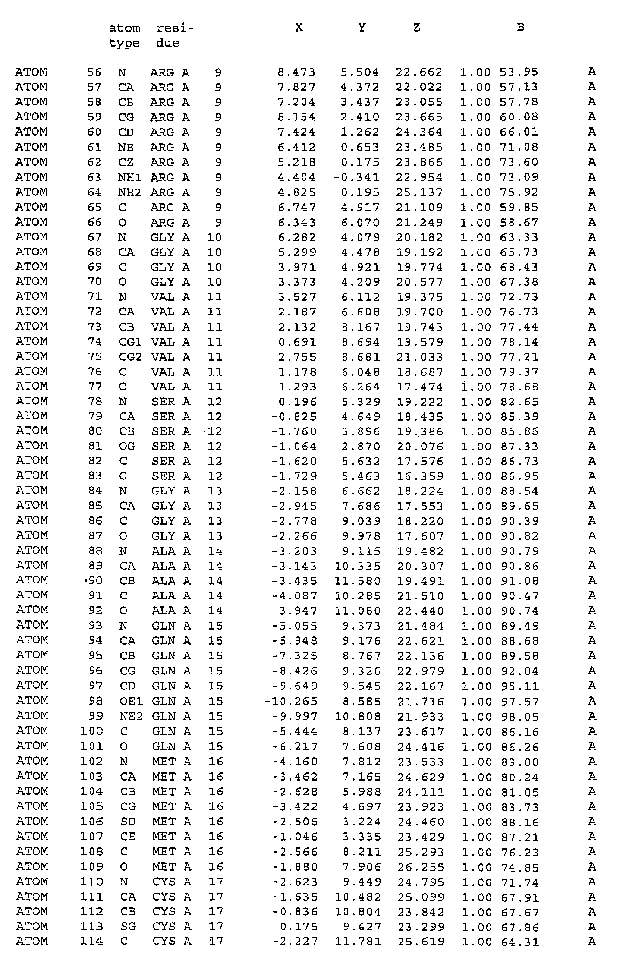

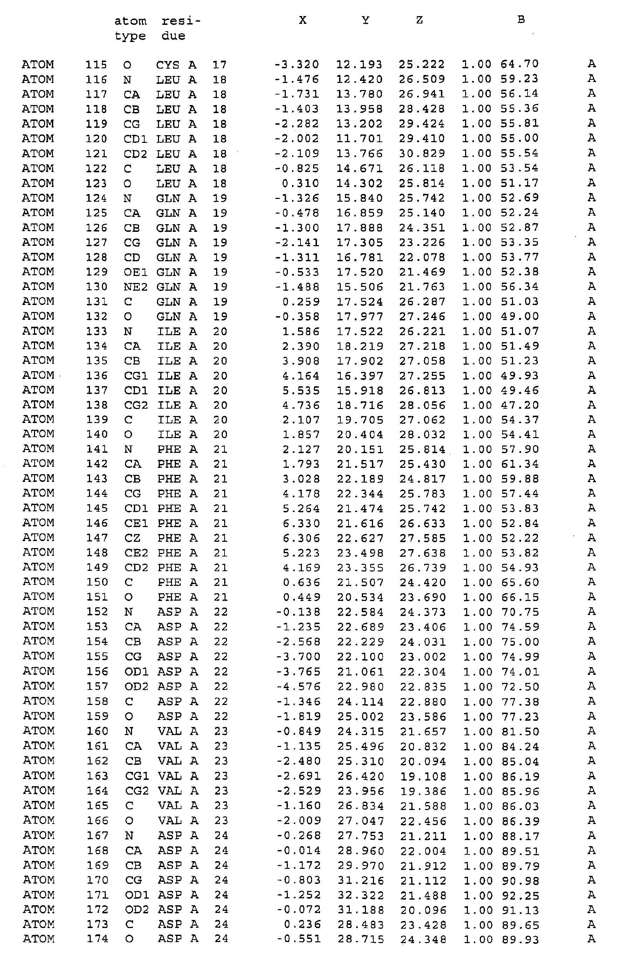

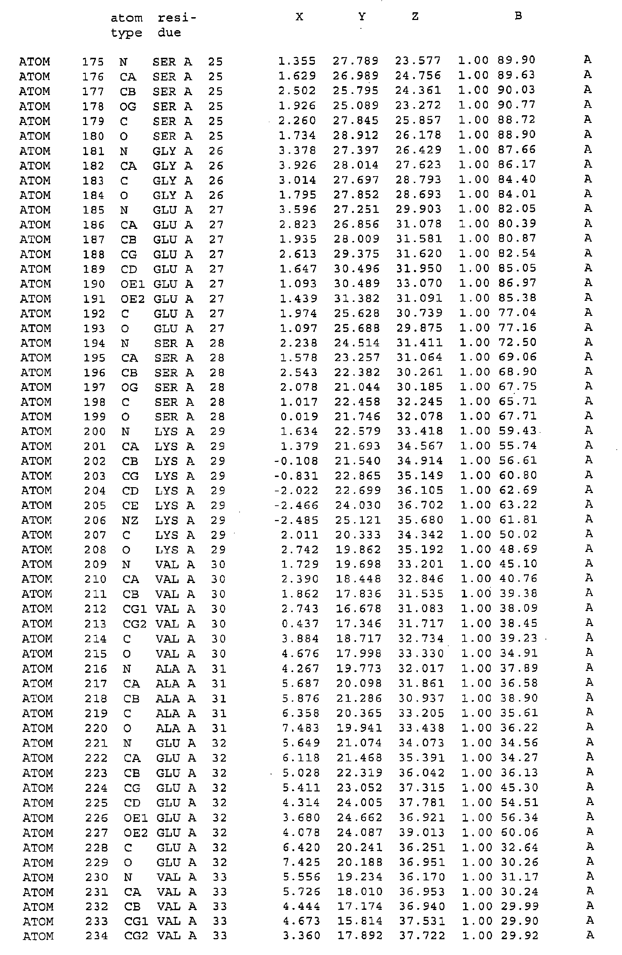

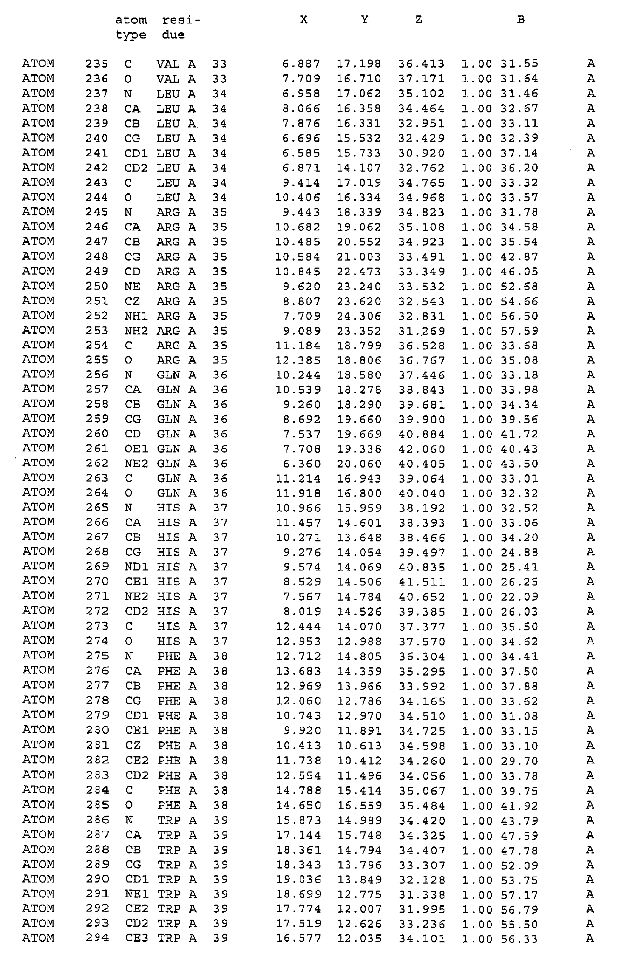

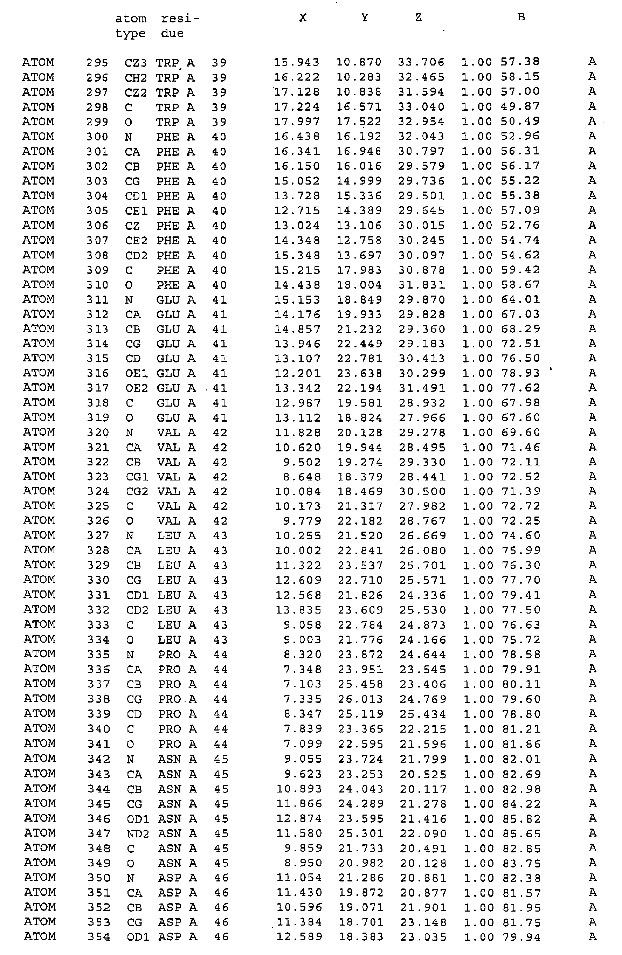

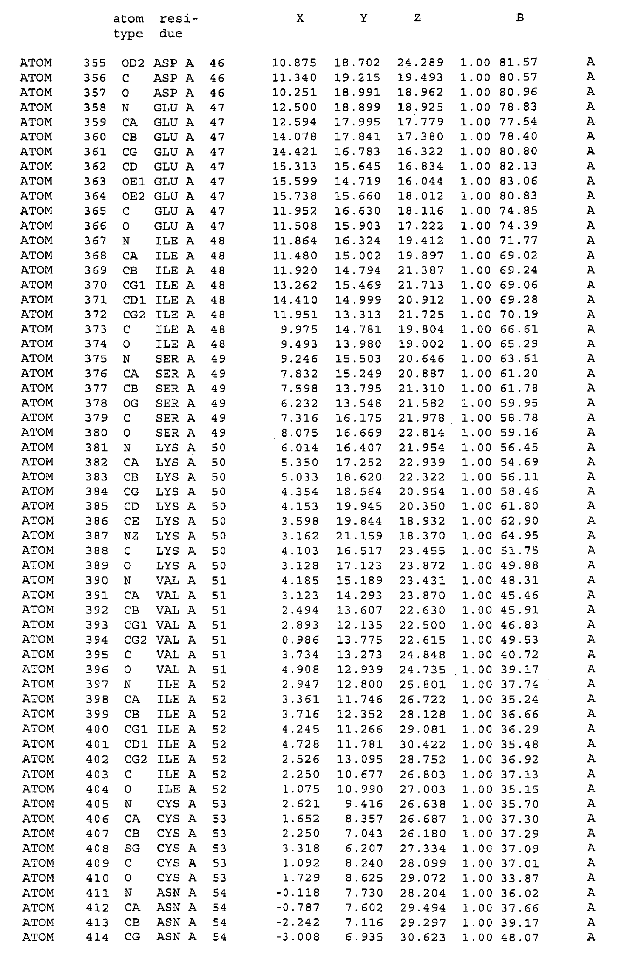

Recent mutant analysis showed, that the proteins encoded by three genes of the fruitfly *Drosophila melanogaster,* grauzone (grau), Zeste-white-5 (Zw5) and Serendipity-δ (Sry-δ) carry vital functions (Chen et al., 2000; Crozatier et al., 1992; Gasner et al., 1999). These proteins all belong to a large group of so-called C₂H₂ zinc finger proteins, which contain zinc ions, which are usually coordinated by two cysteine and two histidine residues. Zinc-finger proteins are defined by the thirty amino-acid C2H2 motive (Miller et al., 1985; Pavlatich and Pabo, 1991). The coordination of a zinc ion by four amino acids plays a crucial role for the functional fold of these proteins and is part of a distinct protein domain, the so-called zinc finger domain. A large number of zinc finger domains function as DNA-binding domains in transcription factors, (for example Pavletich and Pabo, 1991; Rosenberg et al., 1986; Wolfe et al., 2000). Zinc finger domains can also mediate interactions of proteins containing the zinc finger domains with RNA, other proteins and small molecules (Krishna et al., 2003; Makay and Crossley, 1998; McCarty et al., 2003). The zinc finger proteins often contain additional protein domains and primary sequence motifs such as the mammalian Krüppel associated box (KRAB) or the insect zinc finger-associated domain (ZAD) (Lespinet and Aravind, 2002), which can be characterised by a conserved N-terminal primary sequence motif, containing two invariant pairs of cysteines (see for example the primary sequence alignment in Figure 1A). The KRAB domain acts as a transcriptional repressor module (Margolin et al., 1994:; Peng et al., 2000), whereas the function of the zinc finger-associated domain is unknown.

Computer-assisted primary sequence alignments revealed that more than ninety proteins, containing zinc finger-associated domains are present in *Drosophila melanogaster*, among them the above-mentioned Grauzone, Zeste-white-5 and Serendipity-δ (Chung et al., 2002). Grauzone was shown to be necessary for the proper transcriptional activation of the gene cortex, coding for a Cdc20-like APC subunit (Chu et al., 2001). Maternal loss of function mutations of grau cause an irregular growth arrest in meiosis II, whereas homozygous grau mutant embryos, which have received maternal grau activity, develop into normal adults. The mutant phenotype of grau suggests that the transcriptional factor Grauzone is exclusively required during oogenesis and *cortex* is its only target gene (Chen et al., 2000).

The mutational analysis revealing the vital function of Ser-endipity-δ and Zeste-white-5 for *Drosophila melanogaster,* showed that single amino-acid mutations within the respective ZADs of both proteins were responsible for the lethality, implying that conserved amino-acids within the ZAD are essential for the function of these proteins (single amino acid mutations C7T in Serendipity-δ, Crozatier et al., 1992; and R4G in Zeste-white-5, Gazner et al., 1999). These results strongly emphasise the crucial function of ZADs within ZAD-containing zinc finger proteins. ZADs do not exist e.g. in vertebrates and are lineage specific to insects. Therefore ZAD-containing zinc finger proteins might represent novel targets for insecticides.

The utilisation of the ZAD-containing proteins as a target for insecticides is hampered by the fact that no details about the function or the 3-dimensional structure of the ZAD are known. In particular, ZADs were identified as a primary sequence motif by computer-assisted sequence alignments, but up to now it is unclear, if they encompass protein domains with an independent fold, whose 3-dimensional structure can be analysed. Similarly the biological function of ZADs is unknown.

The present invention meets these needs by providing a method for identifying an agent which interacts with a ZAD_{Grau} molecule using the structural coordinates of ZAD_{Grau} according to the base claim 1. Favourable embodiments of the invention are subjects of further claims.

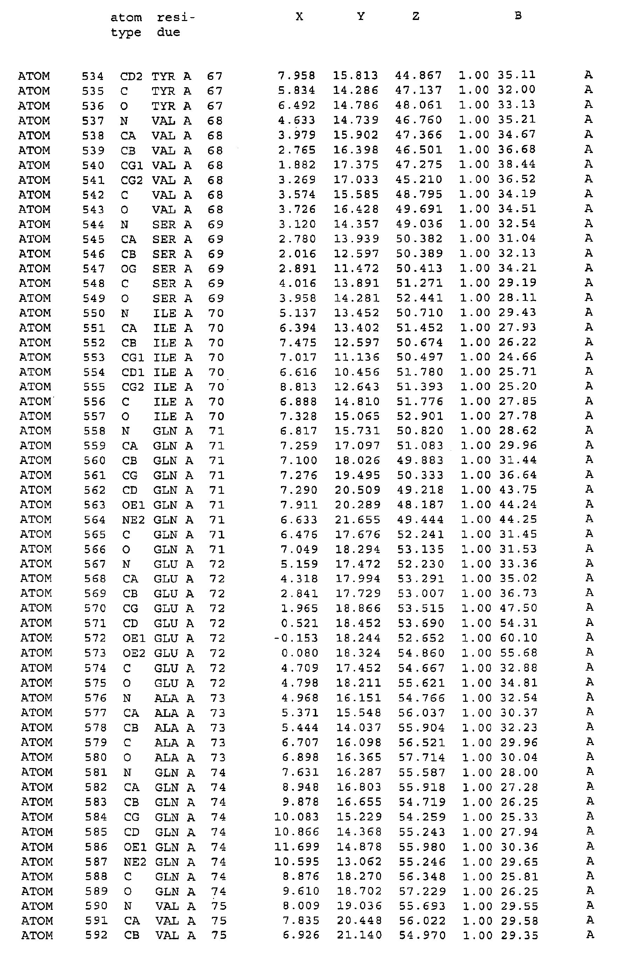

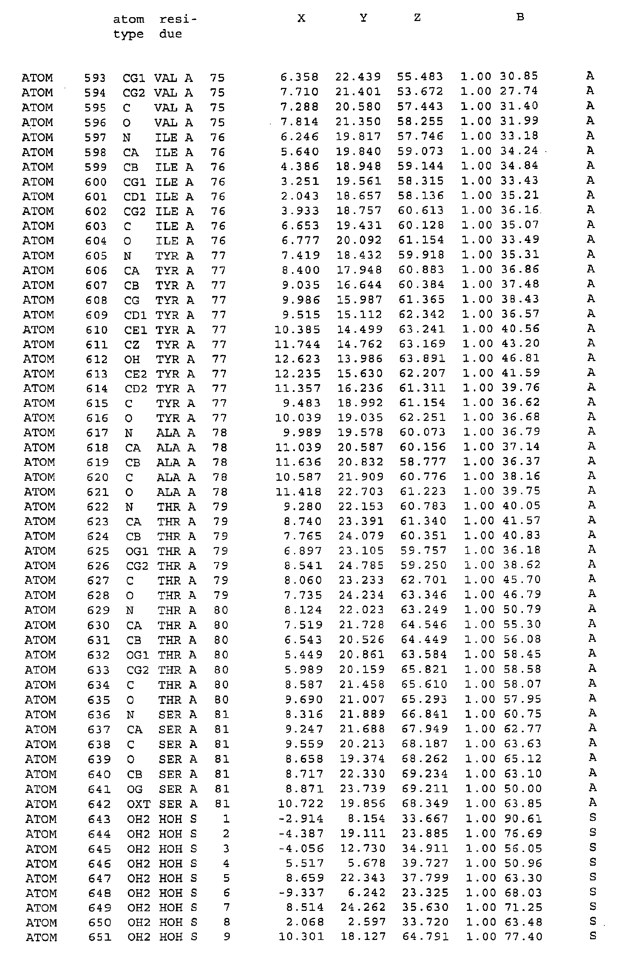

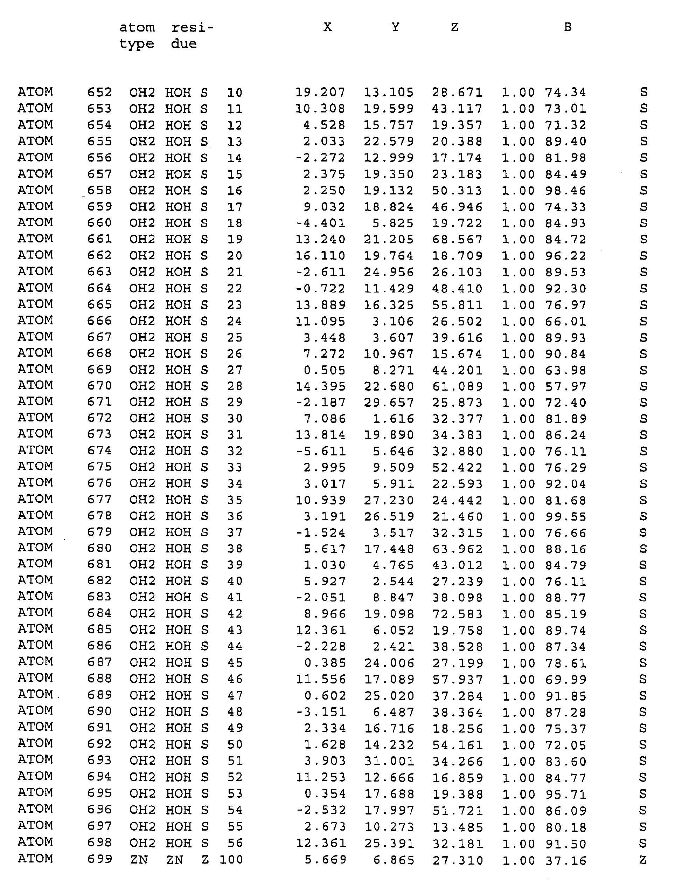

For the first time the invention provides a method for obtaining an agent which interacts with a ZAD_{Grau} molecule or a molecule with a similar fold to ZAD_{Grau}, comprising the following steps:
a) Using the structural coordinates of:
   i) at least a portion of the structural coordinates of ZAD_{Grau} shown in Figure 6 or
   ii) at least a portion of a structural similar molecule of ZAD_{Grau}, having a root mean square deviation from the backbone of ZAD_{Grau} amino-acids of not more than 1.5 Å, to generate a 3-dimensional model of the portion of ZAD_{Grau},
   b) employing the 3-dimensional model and at least one molecular modeling technique to select or design an interacting agent.

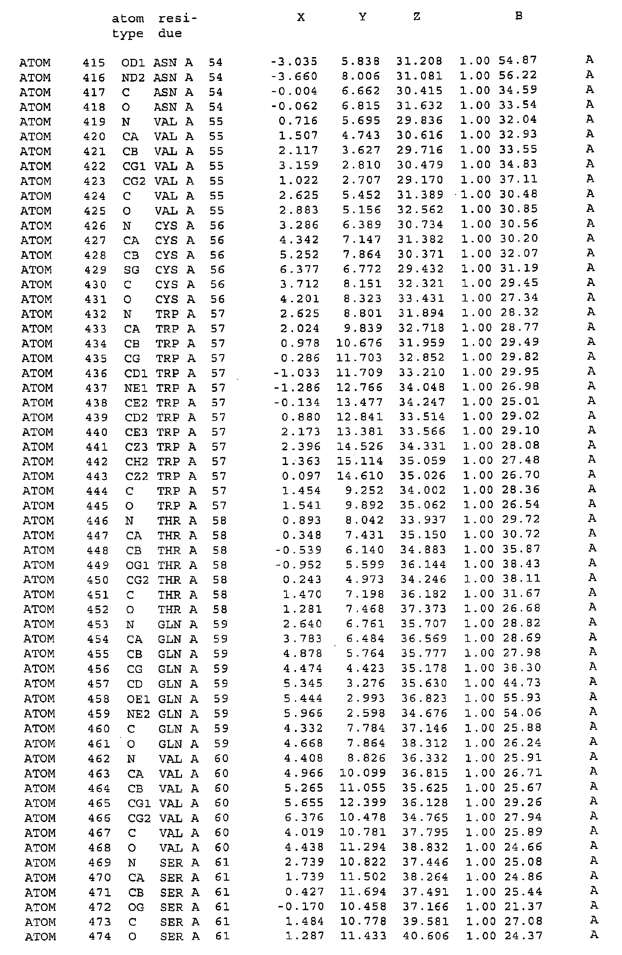

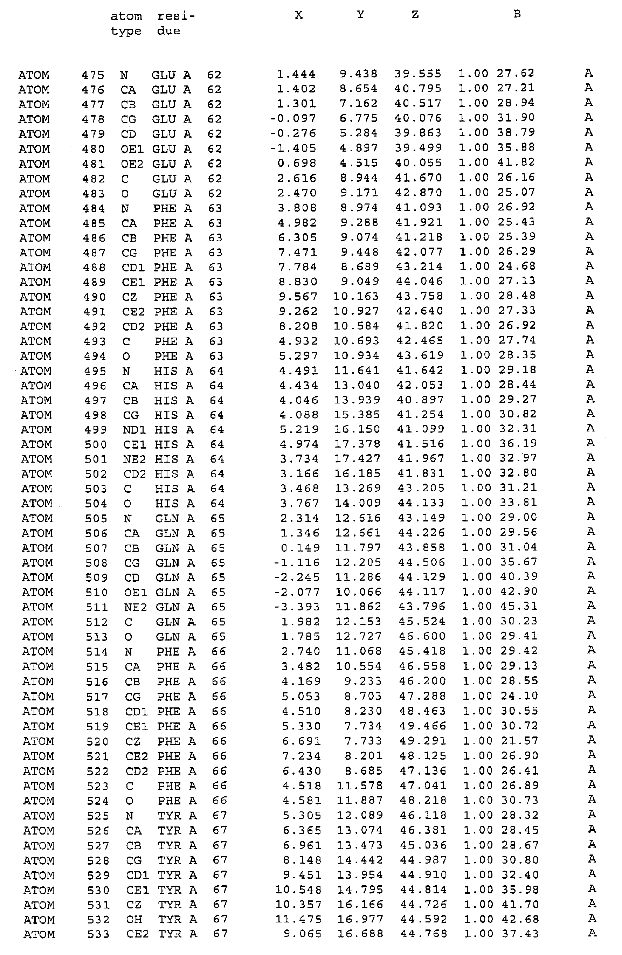

The inventors were, for the first time, able to crystallise isolated ZADs from the protein Grauzone of *Drosophila melanogaster.* A subsequent successful x-ray crystallographic analysis of the protein crystals revealed the 3-dimensional structure of ZAD_{Grau} and showed that ZAD molecules are protein domains with an independent fold, comprising a globular portion structured around a zinc ion at the N-terminus of the protein and a long α-helix at the C-terminus (for details see for example figures 3A to 3C, 4A, 4B and example 3).

In ZAD_{Grau} crystals two ZADs are associated through a 2-fold axis in a head-to-tail fashion, interacting with each other. Major parts of the contact interface are build up by the long α-helix at the C-terminus of the protein (for details see for example figures 4A, 4B and example 3). Therefore the crystal structure unexpectedly suggests that ZAD modules in ZAD-containing proteins function as protein interacting modules, mediating contacts between different proteins e.g. two Grauzone proteins. The dimerization of two ZAD molecules, which was found in ZAD crystals, was also independently confirmed by other biochemical experiments like glutaraldehyde cross-linking experiments and gel filtration experiments in conjunction with multi-angle-laser-light scattering (for details see for example figures 5A, 5B and example 4). The crystallographic data and additional biochemical evidence therefore indicate that ZAD_{Grau} is a protein-protein interaction module with the capability to form homodimers and suggests that ZAD provides a taxon specific means for the assembly of ZAD-containing zinc finger transcription factor complexes.

Consequently the crystal structure of ZADs provides the possibility to obtain agents interacting with ZAD and interfering with the biological function of ZAD, namely the ability to provide protein-protein interactions. Since mutational analysis suggests a vital role of ZADs for insects like *Drosophila melanogaster,* this method can identify potential insecticides.

In the first step a) of the method of the invention at least a portion of the structural coordinates, which were determined by the inventors and are shown in Figure 6, is used to generate a 3-dimensional model of a portion of ZAD_{Grau}. This model can function as a potential binding site for the interacting agent. The knowledge about the exact 3-dimensional geometry of that portion of ZAD is a crucial prerequisite for the selection or the design of agents, interacting with this binding site or interfering with the folding of ZAD. Agents interacting with at least portions of ZAD_{Grau} have to fulfil two requirements. First the agent has to be able to physically and structurally bind to the portion of ZAD_{Grau}. Binding between the agent and the portion of ZAD_{Grau} can occur via salt bridges, hydrogen bonding interactions, hydrophobic interactions or van der Waals interactions. Secondly, the agent must be capable of assuming a conformation that enables association with the portion of ZAD_{Grau} and preferably shows no sterical repulsion.

In step b) of the method of the invention, the 3-dimensional model generated in step a) and at least one molecular modeling technique is used to select or design an interacting agent.

In general molecular modeling involves the computer-assisted calculation, representation and processing of realistic 3-dimensional models of molecules and their physical-chemical properties. Several approaches are possible for the use of the crystal structure of ZAD_{Grau} for identifying interacting agents by molecular modeling techniques. It is possible, for example, to use empirical forcefield methods to describe interactions between atoms in the form of analytical functions an empirical parameters. Using this method it is also possible to calculate binding and nonbinding interactions between atoms. The Insight 11/Discover program package (Accelrys; San Diego, CA) supports different forcefields, for example CVFF (consistent valence forcefield) (Dauber-Osguthorpe et al., 1988), which might help to position potential interacting agents or small chemical entities into binding sites or binding pockets of 3-dimensional portions of the ZAD_{Grau} molecule.

The method of positioning or docking these molecules to binding sites of ZAD_{Grau} may also be accomplished by using software such as Quanta or SYBYL. After the docking operation an energy-minimization using molecular dynamics with standard molecular mechanic forcefields might be useful.

Step b) can further comprise analysing the results of the docking operation to evaluate the interaction between the agent and the portion of ZAD or the portion of the structural similar molecule. This variant of the method of the invention enables the determination of the theoretical binding energy between the agent selected or designed by a molecular modeling technique and the portion of ZAD.

Therefore, computer modeling techniques enable an expert to evaluate the potential binding of agents or small chemical entities to ZAD_{Grau} prior to the actual synthesis of these agents. If the results of the computer modeling techniques suggest insufficient interaction between an agent and ZAD_{Grau}, synthesis and testing of this agent in an assay is omitted.

The positioning of small chemical entities or agents to portions of ZAD_{Grau}, which is also called docking, might also be achieved by other specialized computer programs like:
AUTODOCK (Goodsell, D.S. and A.J. Olsen "Automated Docking of Substrates to Proteins by Simulated Annealing" Proteins: Structure Function and Genetics, 8, pages 195 to 202, 1990).
DOCK (Kuntz, I.D. et al., "A Geometric Approach to Macromolecule-Ligand Interactions", Journal of Molecular Biology, 161, pages 269 to 288, 1982).
GRID (Goodford, P.J. "A Computational Procedure for Determining Energetically Favorable Binding Sites on Biologically Important Macromolecules", Journal of Medicinal Chemistry, 28, pages 849 to 857, 1985).
FLEXX (M. Rary, Kramer B., Lengauer T., Klebe G. "A Fast Flexible Docking Method Using an Incremental Construction Algorithm", Journal of Molecular Biology, 261, pages 470 to 489, 1996). This program uses a special algorithm for docking small chemical compounds into binding sites, using an incremental construction algorithm. Conformational flexibility of the ligands is incorporated by generating many low-energy confirmations of the chemical compounds.

It is also possible to use other molecular modeling techniques. E.g. individual chemical entities which were found to interact with portions of ZAD_{Grau} can also be assembled into a single interacting agent by using for example the following software packages:
CAVEAT (Bartlett, P.A. et al. "CAVEAT: A Program to Facilitate the Structure-derived Design of Biologically Active Molecules", in "Molecular Recognition in Chemical and Biological Problems", special publication of the Royal Chemical Society, 78, pages 82 to 196, 1989).

This program accesses data bases of different compounds which can act as "linkers" in order to connect different chemical entities which were found to bind to structural portions of ZAD_{Grau} .

In accordance with this invention it is also possible to define novel agents in their entirety or by assembly of smaller portions of agents which were selected to bind to ZAD_{Grau} by using for example the software package:
LUDI (Bohm, H.J. "The Computer Program LUDI: A New Method for the De Novo Design of Enzyme Inhibitors", J. Comput. Aided Mol. Des., 6, pp 61 to 78, 1992).

This program uses a big library of different linkers to connect different interaction sites.

According to this invention, molecules which are structurally similar to ZAD_{Grau} have a primary sequence identity of at least 30% to ZAD_{Grau} and form the N-terminal domain of an insect C₂H₂ zinc finger protein. Alternatively structurally similar molecules can be identified by using profile hidden Markow models e.g. with the HMMER software package and the Wise software package as described below.

According to the crystal structure of ZAD_{Grau}, conserved amino acids in ZAD modules can be grouped into three classes: 1. those supporting zinc coordination (C4, C7, C53, C56), 2. those supporting the overall fold, and 3. those supporting dimerization. Class 1 is not an interesting target for ligand design since the coordination of a zinc ion by four cysteines is a common motif in many proteins and not specific to the zinc finger associated domains. Among the second class of residues the sequence H37-V42 is completely conserved, e.g. between *Drosophila melanogaster* and *Anopheles gambiae* ZAD_{Grau} with the exception of F38 *(Drosophila),* which is still conserved as a hydrophobic residue. These residues form a continuous cluster in the globular portion of ZAD_{Grau}, which is well accessible to the solvent and thus suitable for the design of a ligand interacting with this region. In addition, the surface patch outlined by these residues is continuous with that formed by a second cluster of conserved residues, F63-V68 of *Drosophila* ZAD_{Grau}. Several residues in this latter cluster belong to the class 3 of conserved residues, i.e. they mediate important interactions between the molecules forming the ZAD dimers. In particular F63 and F66, which are identical in ZAD_{Grau} from *Drosophila* and *Anopheles,* play important roles in inter-molecular stacking interactions. Again, the surface patch outlined by the latter group of residues is well accessible to the solvent in the ZAD_{Grau} monomer structure. Therefore, the inventors propose to base the design of ligands on the groups of residues H37-V42 and F63-V68 (*Drosophila* numbering). A ligand spanning the two regions or parts of both regions may concomitantly interfere with folding and dimerization of ZADs. Additional residues can be identified in the crystal structure of ZAD_{Grau}, which possibly can be assigned to class 2 as well as class 3, because these residues might be important for the folding of the protein and also support dimerization. These residues are R5, L6, E47, Q59, I70, Q74 and Y77.

The primary sequence identity can be determined using algorithms like Clustal W 1.81 (Thompson et al., 1994). This high percentage of homology suggests an overall 3-dimensional fold of these proteins, which is very similar to ZAD_{Grau}. Experts in the field of protein X-ray crystallography generally accept, that a different set of structural coordinates, e.g. obtained from a different crystal form of the molecule, or crystals of a different ZAD_{Grau} construct (e.g. in combination with other parts of the Grauzone protein), or of a different ZAD alone or in combination with other parts of a protein molecule, still exhibits the same fold as that of ZAD_{Grau} described herein, if the backbone coordinates of the residues comprising the regular secondary structure elements of ZAD-Grau (α-helices and β-strands) do not deviate by more than 1.5 Å. The technical term "root mean square deviation" describes the square root of the arithmetic mean of the squares of the deviation from the mean. It describes the medium variation in the coordinates of the backbone atoms of a protein. Superposition of different sets of structural coordinates can e.g. be performed by using programs like DALI (Holm, L. , Sander, C. Protein structure comparison by alignment of distance matrices. J. Mol. Biol. 233: 123-138, 1993).

In a preferred embodiment of the method of the invention the structural coordinates of at least two of the following ZAD_{Grau} amino-acids shown in Figure 6 are used in step a): R5, L6, E47, Q59, I70, Q74, Y77 and the groups H37-V42 and F63-V68 (sequence numbering corresponding to that of ZAD_{Grau})- It is also possible to use a portion of a structurally similar molecule of ZAD_{Grau}, which has a root mean square deviation from the backbone atoms of the above-mentioned amino-acids of not more than 1.5 Å. The above mentioned amino-acids preferably define the protein-protein interaction site of ZAD_{Grau} or are important for the fold of the ZAD molecule(for details see for example figure 4B or the example 3). Preferably the structural coordinates of at least one of the following amino acids are used in step a.) : arginine 5, phenylalanine 63, phenylalanine 66, isoleucine 70, because these residues are well conserved among the different ZAD molecules (see for example figures 1A and 1B). Agents interacting with some of these amino acids preferably can interfere with the protein-protein interaction properties of ZAD_{Grau} and can therefore present highly valuable insecticides.

In another favorable embodiment of the method of the invention the method further comprises the following steps:
c) Synthesizing said interacting agent and
d) bringing said agent in contact with the binding site of the molecule or structural similar molecule of ZAD to detect the interaction.

These additional steps can provide a valuable verification of the binding of agents, which were selected by computer-based modeling techniques. Step d) can preferably comprise orally administering the interacting agent to flies, due to the ease of handling preferably *Drosophila melanogaster* and detecting the rate of dying or infertile flies in an essay. It is also possible to detect the interaction of the interacting agent with ZAD_{Grau} by e.g. gel filtration chromatography, UV/VIS spectroscopy, fluorescence spectroscopy, ESR spectroscopy, NMR, isothermal titration calorimetrie or "pull down" assays, wherein e.g. a ZAD_{Grau}-glutathione-S-transferase fusion protein is brought in contact with the interacting agent, then precipitated with glutathione-beads and the interacting agent afterwards is detected in the pellet, thereby directly monitoring the interaction between ZAD_{Grau} and the interacting agent.

In a further advantageous embodiment of the method of the invention step b) comprises screening a library of individual chemical entities for the agent by performing a computer-assisted docking operation between the individual chemical entities and the binding site. This can preferably be done by using computer-based libraries of small molecules and by docking, or fitting these molecules into the binding sites of ZAD_{Grau}.

In another embodiment of the invention, step b) comprises the design of the interacting agent by assembling individual chemical entities, which were selected by computer-based modeling techniques to bind to the binding sites of ZAD_{Grau} into a single interacting agent and docking this single interacting agent to the binding site using computational means. This method can be used for "De Novo" synthesis of new interacting agents.

Step b) can further comprise performing an energy minimisation or molecular dynamics operation using computational means to improve the interaction between the agent and the portion of the ZAD molecule or the portion of the molecule with the similar fold. This can be done for example by using the above-mentioned molecular mechanic forcefields to evaluate the best interaction between the agent and the portion of ZAD.

In a preferred variant of the method of the invention, the interacting agent is selected or designed in step b) from a molecule comprising an overall structure similar to ZAD_{Grau}. Since ZAD_{Grau} can interact with itself, putative inhibitors of the biological function of ZAD preferably mimic the overall structure of ZAD_{Grau}.

An agent comprising an overall structure similar to ZAD_{Grau} might exhibit e.g. the same backbone structure as ZAD_{Grau}, or might have functional groups, mimicking amino-acids important for the dimerisation-site, protein-interaction site of ZAD_{Grau} or for the fold of the ZAD_{Grau} molecule. Examples of such amino acids are the above mentioned R5, L6, E47, Q59, I70, Q74, I77 and the groups H37-V42 and F63-V68. Synergetic effects might appear, when residues mimicking both of the above mentioned class 2 and class 3 residues are present in the interacting agent, thereby interfering with the folding of ZAD and the dimerization site.

An interacting agent can therefore be selected or designed from peptidomimetics. These molecules can for example be backbone isosters, having a similar backbone to ZAD_{Grau}. It is also possible to select or design so-called "topographical mimetics", which are non-peptide structures that mimic the binding interactions between two ZAD_{Grau} molecules. These peptidomimetics can comprise non-peptide ligands binding to ZAD_{Grau}, as well as compounds comprising both natural amino-acids as well as non-natural elements for example non-natural amino-acids. The peptidomimetics can therefore be compounds with structures in between a true peptide and a complete peptide mimetic, which entirely consists of non-natural elements. Methods of designing peptidomimetics, mimicking the structure of natural peptide compounds, are for example disclosed in the following publications, which are hereby incorporated in their entirety:
Hruby, V.J., Balse, P.M., "Conformational and Topographical Considerations in Designing Agonist Peptidomimetics from Peptide Leads", Current Medicinal Chemistry, 7, pp 945 to 970 (2000) .
Kieber-Emmons, T., Murali, R., Greene, M.I. "Therapeutic Peptides and Peptidomimetics", Current Opinion in Biotechnology, 8, pp 435 to 441, (1997).
Wiley, R.A., Rich, D.H., "Peptidomimetics Derived from Natural Products", Medicinal Research Reviews, 13, pp 327 to 384, (1993) .
Kemp, D.S., "Peptidomimetics and the Template Approach to Nucleation of Beta-Sheets and Alpha-Helices in Peptides", Trends in Biotechnology, 8, pp 249 to 255 (1999).

Alternatively the interacting agent is selected or designed by linking functional groups mimicking important side chains for the dimerization of ZAD_{Grau} by so-called spacer groups. These spacer groups could comprise the already above-mentioned "linkers" which for example are a part of the software package LUDI.

Yet another object of the invention is a method of obtaining a 3-dimensional model of a ZAD_{Grau}-like molecule of unknown structure, comprising the following step:
A1) using
   - computational means,
   - structural and/or sequence information of the ZAD_{Grau}-like molecule of unknown structure and
   - at least a portion of the structural coordinates of the ZAD_{Grau} molecule shown in Figure 6,
   to obtain the 3-dimensional model of the ZAD_{Grau}-like molecule.

This method of the invention is very useful for the determination of the 3-dimensional structures of ZAD_{Grau}-like molecules, which exhibit a high primary sequence homology to ZAD_{Grau} and therefore exhibit a similar 3-dimensional fold. Structural information about the ZAD_{Grau}-like molecule of unknown structure can for example be obtained in the form of x-ray reflection intensities from crystals of the ZAD_{Grau}-like molecule. It is also possible to use primary sequence information of the ZAD_{Grau}-like molecule in this method of the invention.

ZAD_{Grau}-like molecules of unknown 3-dimensional structure preferably exhibit a primary sequence similarity (homology) of at least 30%. ZAD_{Grau}-like molecules comprise short around 90 amino acid protein modules found as N-terminal domains of C₂H₂ zinc finger proteins in insects. They contain four invariant cysteine residues.

ZAD_{Grau}-like molecules can preferably also be identified by using e.g. the HMMER package 2.1.1 (Eddy, S.R. (1998) Profile hidden Markov models. *Bioinformatics,* 14, 755-763) to construct a profile hidden Markow model (HMM). Data obtained from the HMM can then be used to search DNA-databases employing e.g. the Wise package 2.2.0 (Birney E., Thompson J.D. and Gibson T.J. (1996) PairWise and SearchWise: finding the optimal alignment in a simultaneous comparison of a protein profile against all DNA translation frames. Nucleic Acids Res. 24(14):2730-9).

Preferably the primary sequences of the proteins are aligned for maximum homology. Homologous protein variants comprise a large number of matching amino-acids, which can be aligned and have similar structural or chemical properties. In general similar amino acids can be divided into different groups depending on the nature of their side chains. Similar amino acids having basic side chains comprise for example lysine, arginine, histidine. Similar amino-acids with acidic side chains comprise for example aspartic acid or glutamic acid. Amino-acids with uncharged polar side chains are, example given, glycine, asparagine, glutamine, serine, threonine, tyrosine or cysteine. Similar amino-acids with non-polar side chains are for example alanine, valine, leucine, isoleucine, proline, phenylalanine, methionine or tryptophane. Similar amino-acids with aromatic side chains are e.g. tyrosine, phenylalanine, tryptophane or histidine. In homologous primary protein sequences large regions of similar amino-acids which can be aligned, are often interrupted by regions where non-conservative changes occur, for example replacement of a leucine with an aspartic acid. Further, homologous protein variants may also include amino-acid deletions or insertions or both.

Preferably step A1) of the method of the invention comprises the following steps:
A11) Obtaining x-ray diffraction data of a crystallised ZAD_{Grau}-like molecule,
A12) using the portion of the structural coordinates of the ZAD_{Grau} molecule to obtain an electron density map of the ZAD_{Grau}-like molecule, and
A13) building of a 3-dimensional model of the ZAD_{Grau}-like molecule into the electron density map.

This method uses x-ray diffraction data from a ZAD-like molecule of unknown 3-dimensional structure and e.g. molecular replacement techniques in order to obtain a 3-dimensional model of the ZAD_{Grau}-like molecule of unknown structure.

Molecular replacement methods involve the computer-assisted calculation of patterson vector maps, which show the vectorial distances between atomic positions in the 3-dimensional structures. During the process of molecular replacement rotation and translation of this patterson map is performed within the unit cell of the crystal of unknown 3-D structure in order to superimpose the known 3-dimensional structure of ZAD_{Grau} onto the ZAD_{Grau}-like molecule. Software packages for molecular replacement are for example programs like Molrep or AmoRe (A.Vagin, A.Teplyakov, MOLREP: an automated program for molecular replacement., J. Appl. Cryst. 30, 1022-1025 (1997) and J. Navaza, AMoRe: an automated package for molecular replacement Acta Cryst. A50, 157-163 (1994)). A more detailed description of molecular replacement is given in chapter 10 "Molecular Replacement" in Drenth, "Principles of Protein X-Ray Crystalography", 1994, Springer, pp 213 to 234, which is hereby incorporated in its entirety.

By using these molecular replacement methods a preliminary electron density map of the ZAD_{Grau}-like molecule of unknown 3-dimensional structure can be obtained. A 3-dimensional model of this ZAD_{Grau}-like molecule can then be built in step A13) of the method of the invention into the electron density map. This often involves the building of a preliminary, for example, polyalanine model into the electron density map and then iterative manually or computer-assisted building and refinement of additional elements, e.g. amino acid side chains in order to obtain a more accurate model of the ZAD_{Grau}-like molecule.

In an alternative method step A1) can also comprise using a homology modeling program and the primary sequence of the ZAD_{Grau}-like molecule in order to obtain the 3-dimensional model of the ZAD_{Grau}-like molecule.

In general homology modeling uses known primary sequence information and also known structural information in order to predict an unknown 3-dimensional structure of a protein. Software packages for homology modeling of proteins comprise for example SYBYL or Swiss model (Tripos, St. Louis, MO; SWISS-MODEL: an automated protein homology-modeling server. Nucleic Acids Res. (2003) 31:3381-3385).

During the molecular replacement process or homology modeling process a primary sequence alignment between ZAD_{Grau} and the ZAD_{Grau}-like molecule with the unknown 3-dimensional structure might be generated. As mentioned above this involves the alignment of matching homologous amino acids. For example it is possible to adjust the 3-dimensional model of ZAD_{Grau} according to the primary sequence alignment between ZAD_{Grau} and the ZAD_{Grau}-like molecule whose 3-dimensional structure has to be determined. This can be done for example by changing the amino acid side chains of the 3-dimensional model of ZAD_{Grau}, for example to alanine where non-conservative changes occur. This model can then be used e.g. as a search model for the rotation and translation searches of the molecular replacement method.

Using the molecular replacement method or the homology modeling method of the invention, the 3-dimensional structures of ZAD_{Grau}-like molecules for example from *Anopheles gambiae* or from other ZADs of *Drosophila melanogaster* can be determined. In general the 3-D structures of ZAD_{Grau}-like molecules having a sequence homology of at least 30 % to ZAD_{Grau} can be determined with this method of the invention.

Since the inventors revealed, that ZADs contain a zinc ion, coordinated by four invariant cysteines, it is also possible to determine the structure of ZAD_{Grau}-like molecules of unknown 3-dimensional structure by multiple wavelength anomalous dispersion (MAD) using the anomalous scattering of this zinc-atom of ZADs. MAD is described e.g. in chapter 9 "Anomalous Scattering in the Determination of the Protein Phase Angels and the Absolute Configuration" in Drenth, "Principles of Protein X-Ray Crystallography", 1994, Springer, pp 201 to 212, which is hereby incorporated in its entirety.

Another embodiment of the invention are crystals comprising. ZAD molecules. These crystals preferably diffract x-rays to a resolution of at least 3 Å and can be used to determine the 3-dimensional structures of the ZAD molecules in the crystal. Preferably the crystal comprises ZAD_{Grau} molecules with the structural coordinates shown in Figure 6 or structurally similar molecules of ZAD_{Grau} comprising structural coordinates with a root mean square deviation from the backbone atoms of the coordinates shown in Figure 6 of not more than 1.5 Å.

According to another preferred embodiment of the invention a computer system for generation and computational utilization of a 3-dimensional model of ZAD is provided, comprising the following components:
I) A computer-readable medium having stored thereon
   i) at least a portion of the structural coordinates of all the ZAD_{Grau} amino-acids shown in Figure 6 or
   ii) at least a portion of a structurally similar molecule of ZAD_{Grau}, having a root mean square deviation from the backbone of the ZAD_{Grau} amino-acids of not more than 1.5 Å, or
   iii) x-ray diffraction data of ZAD_{Grau} or a structurally similar molecule of ZAD_{Grau}, having a root mean square deviation from the backbone of the ZAD_{Grau} amino-acids of not more than 1.5 Å,
II) computing subroutines for the generation and computational utilization of the said structural coordinates, and
III) a central processing unit for performing said computing subroutines.

Such a computer system is useful for analyzing the x-ray diffraction intensities of crystals comprising ZAD molecules. The computer system of the invention can also be used for the determination of the 3-dimensional structure of ZAD_{Grau}-like molecules by using the 3-dimensional coordinates of ZAD_{Grau} by using e.g. molecular modeling or molecular replacement subroutines.

The computing subroutines of component II) are preferably selected from a group consisting of x-ray data processing, reduction, indexing, merging, intensity scaling and truncation. The subroutines are for example included in programs like DENZO, MOSFLM, the CCP4-package or X-PLOR. Subroutines can also include programs for molecular replacement and molecular modeling, for example the above-mentioned SYBYL, Swiss model, AmoRE or molrep.

In the following the invention will be explained in more detail by the figures and the embodiments.

Figure 1 depicts a primary sequence alignment between the ZAD from Grauzone, its closest paralogue CG15073 in the *Drosophila melanogaster* genome and an *Anopheles gambiae* homologue.

Figure 1B shows a primary sequence alignment between ZAD_{Grau} and 32 other homologous proteins from *Drosophila melanogaster* and *Anopheles gambiae.*

Figures 2A to 2C depict biochemical and biophysical experiments revealing that ZAD_{Grau} comprises a zinc coordinating fold and an electron density map around the zinc center in ZAD_{Grau}.

Figures 3A to 3C show the 3-D structure and topology of ZAD-Grau.

Figures 4A and 4B show stereo ribbon plots of ZAD_{Grau} dimers in the crystal.

Figures 5A and 5B depict additional biochemical experiments showing that ZAD_{Grau} dimerizes in solution.

Figure 6 shows the structural coordinates of ZAD_{Grau}.

### Example 1: Cloning of ZAD_{Grau} and Sample preparation:

The DNA coding for Grauzone ZAD residues 2-90 was PCR-amplified, cloned into the Not1/EcoR1 sites of the pGEXT-3 vector and recombinantly expressed as a GST-fusion protein in a BL21(DE3) *E. coli* strain. Cells were grown to an OD600 0.7-1.0 at 30°C, shifted to 20°C and induced with 0.7 mM IPTG over night. Cells were harvested by centrifugation and resuspended in phosphate-buffered saline containing Complete™ protease inhibitor tabs (Roche) and traces of lysozyme. Cells were lysed by sonication and the insoluble fraction was removed by centrifugation. GST-ZAD_{Grau} was captured using Glutathione (GSH)-Sepharose 4B (Amersham) beads in disposable columns following the manufacturer's suggestions. Samples were eluted with 50mM Tris-HCl, pH 8.0, 50mM GSH. Thrombin was directly added to the eluate to 1 unit/100µg protein and incubated overnight at room temperature. The protein identity was confirmed by peptide finger printing and subsequently purified with a 1ml Ressource-Q 15µm anion exchange column applying a linear 20ml salt gradient (20mM Tris-HCl, pH 8.0, 0-1M NaCl), a second GSH-column and a Superdex 25/60 75pg gel filtration (GF) column (GF buffer: 10mM Tris pH 7.5, 100mM NaCl, 5mM DTT). Samples were concentrated to 8mg/mL via 20ml 5000Da molecular weight cutoff concentrators (Vivascience).

### Example 2: Crystallisation of ZAD_{Grau}, X-ray fluorescence spectra and data collection:

Crystallization screens were carried in the sitting drop vapor diffusion format on 24-well Cryschem plates™ (Hampton Research, Laguna Niguel, USA). 1-4µl of an 8mg/ml ZAD_{Grau} solution in GF buffer were combined with 1µl reservoir buffer and equilibrated against a 400µl reservoir. Crystals grew at 22°C with 0.2M ammonium acetate, 0.1M sodium citrate, pH 5.6, and 30% polyethylene glycol 4000 in 3-10 days.

After transfer into Paratone-N (Hampton Research) and removal of residual mother liquor, crystals could be shock frozen in a liquid nitrogen stream. Diffraction data were collected at 100K on the HASYLAB beamline BW6 (DESY, Hamburg, Germany; http://www-hasylab.desy.de/facility/experimental_stations/stations/BW6.htm).

X-ray fluorescence spectra of native crystals clearly indicated zinc emission lines. Figure 2B depicts a X-ray emission spectrum of a native ZAD_{Grau} crystal irradiated with X-radiation of λ = 1.0Å. The peaks in the X-ray emission spectrum are denoted as follows: I - ZnKα line; II - ZnKβ line; III - Compton scattering; IV - Elastic scattering. A single ZAD_{Grau} crystal yielded complete anomalous data sets at the f' '-maximum of the K-edge (λ = 1.2828Å) and at a remote wavelength (λ = 1.0500Å). The data were recorded on a MarResearch (Norderstedt, Germany) CCD detector and processed with the HKL package ((Otwinowski and Minor, 1997). The table shows the crystallographic data.

**Table:**

| Crystallographic Data | | | |
|---|---|---|---|
| **Dataset** | | **Remote** | **Peak** |
| ***Data Collection*** | | | |
| **Spacegroup** | | P4₁2₁2 | |
| **Unit Cell Lengths (Å)** | a | 48.7 | |
| | b | 48.7 | |
| | c | 82.1 | |
| **Wavelength (Å)** | | 1.0500 | 1.2828 |
| **Resolution (Å)** | | 50.0-2.0 | 50.0-2.5 |
| **Unique Reflections** | | 6907 | 6089 |
| **Redundancy** | | 4.6 | 2.6 |
| **Completeness (%)** | | 97.0 (99.7) | 96.3 (98.5) |
| **I/σ(I)** | | 36.7 (6.3) | 28.2 (14.2) |
| **R**_{**sym**}^{**a**} **(%)** | | 4.2 (44.7) | 3.1 (8.2) |

| ***Phasing*** | | | |
|---|---|---|---|
| **Resolution (Å)** | | 20.0-2.1 | |
| **Heavy Atom Sites** | | 1 | |
| **R**_{**cuilis**}^{**b**} | Centrics | 0.50 | |
| | Acentrics | 0.61 | |
| **Phasing Power**^{**c**} | Centrics | 1.67 | |
| | Acentrics | 1.97 | |
| **FOM**^{**d**} | Before DM^{e} | 0.42 | |
| | After DM | 0.78 | |

| ***Refinement*** | | | |
|---|---|---|---|
| **Resolution (Å)** | | 15.0-2.0 | |
| **Number of Reflections** | | 6590 | |
| **Number of Non-Hydrogen Atoms** | | | |
| Protein | | 644 | |
| Zn²⁺ Ions | | 1 | |
| Water Oxygens | | 56 | |
| **R**_{**work**}^{**f**} **(%)** | | 24.1 (29.6) | |
| **R**_{**free**}^{**f**} **(%)** | | 26.7 (33.7) | |
| **RMSD**^{**g**} **From Ideal Geometry** | | | |
| Bond Lengths (Å) | | 0.009 | |
| Bond Angles (°) | | 1.20 | |
| **Average B-Factors (Å**^{**2**}**)** | | | |
| Protein | | 50.6 | |
| Zn²⁺ Ion | | 37.2 | |
| Water Oxygens | | 80.3 | |
| Wilson B-Factor | | 52.6 | |
| **RMSD B-Factors (Å**^{**2**}**)** | | | |
| Main Chain Bonds | | 1.9 | |
| Main Chain Angles | | 3.3 | |
| Side Chain Bonds | | 3.5 | |
| Side Chain Angles | | 5.2 | |
| **Ramachandran Analysis (%)** | | | |
| Preferred | | 91.9 | |
| Additionally Allowed | | 6.8 | |
| Disallowed | | 1.4 | |
| **Mean Residual Error (Å)** | | 0.15 | |

| | | | |
|---|---|---|---|
| ^{a} R_{sym}(I) - = (∑ₕₖₗ∑ᵢ[\|*I*ᵢ(hkl) - <*I*(hkl)> ] / ∑ₕₖₗ∑ᵢ [*I*ᵢ (hkl)]\|; Iᵢ(hkl) - intensity of the i^{th} measurement of hkl; <*I*(hkl)> - average value of hkl for all i measurements | | | |
| ^{c} R_{cullis} = ∑ₕₖₗ[\|\|*F*_{PH} ± *F*_{P}\| - \|*F*_{H,calc}\|] /∑ₕₖₗ [\|*F*_{PH} ± *F*_{P}\|] ; (*F*_{PH} + *F*_{P})if signs are opposite, (*F*_{PH} - *F*_{P}) if equal | | | |
| ^{c} Phasing Power = (∑ₙ [\|*F*ₕ\|²] / ∑ₙ[\|*E*\|²])^{1/2}; ∑ₙ\|*E*²\| = lack of closure error = ∑ₙ[\|*F*_{PH}\| (obs) - \|*F*_{PH}\| (calc) ]² | | | |
| ^{d} FOM = Figure of merit = [\|*F*(hkl) _{best}\|] / \|*F*(hkl)\| ; **F**(hkl)_{best =} ∑_{α}[*P*(α)**F**ₕₖₗ(α)] / ∑_{α}[*P*(α)] | | | |
| ^{e} DM - density modification (solvent flattening) | | | |
| ^{f} R_{work} = ∑ₕₖₗ [\|\|F_{obs}\| - *k*\|F_{calc}\|\|] / ∑ₕₖₗ [\|F_{obs}\|] ; R_{free} = ∑_{hkl⊂T}[\|\|F_{obs}\| - k\|F_{calc}\|\|] / ∑_{hkl⊂T} [\|F_{obs}\|] ; hkl⊂T - test set | | | |
| ^{g} RMSD - root-mean-square deviation Data for the last 0.05Å in parentheses | | | |

### Example 3: Structure solution of ZAD_{Grau}, model building and refinement:

If not mentioned otherwise, programs from the CCP4 collection (Project., 1994) were employed for the structure solution. The location of a single Zn²⁺ ion could be deduced from anomalous difference Patterson maps at the f' ' -maximum (program RSPS) or by direct methods (SHELX, http://shelx.uniac.gwdg.de/SHELX/) and was used to phase the data with the remote data set as the reference (MLPHARE). The hand of the heavy atom was revealed during solvent flattening (DM). Prominent secondary structure elements were manually fitted to the electron density with MAIN (Turk, 1996). Subsequently, these regions were decorated with side chains guided by the prominent features of the aromatic residues and by four cysteine residues, which coordinated the Zn²⁺ ion. The connecting loops were sequentially traced by alternating refinement of the partial model (CNS) (Brunger et al., 1998) and model building guided by the experimental and 2Fₒ-F_{c} electron density maps. Refinement included standard procedures of positional and B-factor optimization, a round of simulated annealing and positioning of 56 water molecules into spherical peaks of the Fₒ-F_{c} maps. The final step was a TLS refinement with REFMAC5 with the structure divided into four rigid bodies (the Zn²⁺ ion, residues 2-19 and 51-54, residues 20-50, and residues 55-81). No data within 15.0-2.0Å were excluded and during all refinement steps the same set of reflections (5%) was used to monitor the R_{free}-factor (see Table). The geometry of the final model was analyzed with PROCHECK ((Laskowski et al., 1993); see Table), secondary structure elements were extracted with PROMOTIF (http://www.biochem.ucl.ac.uk/bsm/promotif/promotif.html).

### ZAD is a Zinc-Binding Protein Module

Based on the four conserved cysteine residues, ZADs have been suggested to comprise metal-binding modules (Chung et al., 2002; Lander et al., 2001; Lespinet and Aravind, 2002). Because metal association is expected to contribute significantly to the stability of the fold, heat denaturation experiments were performed in the presence and absence of EDTA. In the absence of EDTA, ZAD_{Grau} was stable for at least 15 minutes at 60°C, whereas in the presence of EDTA (5mM) a considerable portion of the protein precipitated when heated above 50°C for 15 minutes (see Figure 2A). In these experiments care was taken to exclude divalent metal ions from the purification procedure. The result therefore suggests that ZAD_{Grau} contains an endogenous metal center that confers stability on its structure. For EDTA-dependent precipitation studies, 10µM ZAD_{Grau} samples in GF buffer either lacking EDTA or containing 5mM EDTA were incubated for 15min at room temperature, 37°C, 50°C and 60°C. Samples were pelleted and the supernatant was analyzed by SDS-PAGE.

Figure 2A shows a denaturing SDS-PAGE with the portion of ZAD_{Grau} remaining soluble after the above mentioned EDTA-dependent precipitation studies. The samples were incubated at the indicated temperatures in the absence (-EDTA) and in the presence of 5mM EDTA (+EDTA). Reduction of the soluble fraction is clearly visible when ZAD_{Grau} is heated above 50°C in presence of the chelator EDTA.
In order to test the proposal, that ZAD_{Grau} contains a zinc metal center, the inventors performed an X-ray fluorescence scan. With this approach, the characteristic emission lines for zinc were observed (see Figure 2B and example 2). This result and the finding that the anomalous diffraction from the zinc center could be exploited to solve the structure with a native crystal (see below) demonstrate that ZAD_{Grau} includes a zinc ion.

### Structure Solution and Quality of the Model

The primary sequence of ZAD_{Grau} is shown in Figure 1A. This Figure 1A shows an alignment of the Grauzone ZAD with its closest paralog, CG15073, in the *Drosophila melanogaster* genome and with a putative *Anopheles gambiae* homolog. Asterisks mark 100% conservation in all three ZADs. The grey boxes show conserved hydrophobic or aromatic amino acid residues. The secondary structure elements of ZAD_{Grau} are shown as arrows (β-sheets) or tubes(α-helices).

The crystal structure of ZAD_{Grau} was solved de *novo* by a two-wavelength multiple anomalous dispersion experiment around the K-edge of an endogenous Zn²⁺ ion. A segmented polyalanine model could be manually fitted to the experimental electron density map and allowed the subsequent incorporation of side chains. The sequence assignment was guided by well-defined aromatic residues and by the two pairs conserved cysteines (C4, C7, C53 and C56; see Figures 1A, 2C), which coordinated the Zn²⁺ ion.

Figure 2C shows a stereo plot of the electron density maps around the zinc center in ZAD_{Grau}. The experimental MAD electron density map (1 sigma) is shown around the zinc-coordinating cysteines. Around the Zn²⁺-ion an anomalous difference Fourier map (10 sigma), generated with the anomalous differences at the peak wavelength and the phases obtained after solvent flattening is shown. The zinc-coordinating cysteine residues are labeled and shown in ball-and-stick. If not mentioned otherwise, all structural figures were prepared with Bobscript (http://www.strubi.ox.ac.uk/bobscript/) and rendered with Raster3D (Merritt and Bacon, 1997)

Three regions in flexible loops (V11-C17, D22-E27 and E41-E47; see Figures 1A, 3A) initially displayed weak densities and were fitted during the subsequent rounds of refinement and manual model building. These loop regions, which are made up of non-conserved residues, lack substantial intra- and interchain contacts. Thus, they gained above-average temperature factors during the refinement. The main chain electron density of the molecule remained fragmented around position S12-A14 and D22 in the final 2Fₒ-F_{c} map, consistent with high internal flexibility and lack of crystal packing contacts in these regions. All other side chains of the refined model, except those of some hydrophilic surface residues, were entirely covered by the final 2Fₒ-F_{c} map. No electron density developed for the C-terminal nine residues (positions 82-90; Figure 1A) of ZAD_{Grau} and the five N-terminal vector-derived residues that are contained in the recombinant protein. The final model therefore encompasses the residues D2-S81 of ZAD_{Grau} that could be traced unambiguously. Presumably because of the considerable fraction of flexible residues at the termini, which could not be accounted for in the final model, the refinement converged with an R-factor/R_{free}-factor of 24.1%/26.7%. Convergence at these numbers is consistent with a rather high B-factor extracted from a Wilson plot (52. 6Å²), which is comparable to the averaged B-factor of the final structure (52.9Å²) .

During all refinement steps 5% of the reflections were set aside to monitor the R_{free}-factor (see Table) . Of the final model, 91.9% of the residues resided in the preferred regions of the Ramachandran plot, 6.8% in the additionally allowed areas. Only the single residue K29, which was well defined in both the experimental and the final model-derived maps assumed an unconventional φ/ψ conformation even after manual interference. The mean positional error of the model was estimated at 0.15Å (Luzzati, 1952).

### Structural Properties of ZAD_{Grau}

The structured portion of ZAD_{Grau} resembles the letter 'b'. Its approximate dimensions are 60Å x 30Å x 30Å (Figure 3A and 3B). Figure 3A shows a topological diagram of the ZAD_{Grau} structure identifying the origin of the four zinc-coordinating cysteines and the borders of the secondary structure elements (numbers). The Figures 3B and 3C depict two orthogonal stereo ribbon plots of the ZAD_{Grau} structure. In the side view of Figure 3B the structure resembles the letter 'b'. N- and C-termini, secondary structure elements and residues of the zinc center are labeled. The Zn²⁺ ion and its coordinating cysteine side chains are shown in ball-and stick.

The N-terminal body of the 'b' comprises a globular fold structured around a zinc ion. The C-terminal stem is formed by a long α-helix (α2, positions 54-80) that contains almost one third of all residues of the domain. Residues 82-90 are not included in the ZAD consensus sequence (Chung et al., 2002) and are disordered in the present structure. Presumably, they provide a flexible linker to the remainder of the molecule, i.e. an acidic region followed by an array of eight C₂H₂ zinc fingers in the case of Grauzone (Chen et al., 2000) .
The arrangement of the different ZAD structural elements is summarized in Figures 3A-3C. It shows that the N-terminus of ZAD_{Grau} is folded into a hairpin motif (D2-V11; Figure 3A-3C). It contains two zinc-coordinating cysteine residues (C4 and C7; Figure1A), which are conserved in all ZADs so far identified (Chung et al., 2002). Replacement of C7 by a tyrosine residue has been described as a partially lethal mutation of Sry-δ (Crozatier et al., 1992). This observation implies that C7 is essential for ZAD function and supports the argument that C7 is directly involved in zinc coordination. The N-terminal hairpin is followed by a loop (L1: S12-M16) extending into a two-stranded antiparallel β-sheet (β1: C17-Q19, β2: V51-C53). The β-sheet is interspersed by a long loop-helix-loop insertion (L2: I20-K29, α1: V30-H37, L3: F38-K50). The C-terminal helix, α2, is directly linked to the second β-strand with a slanting angle of ∼80° between the helix axis and the average long axis of the β-sheet. Helix α1 of the L2-α1-L3 motif is positioned perpendicular to both the long axis of the sheet and the axis of helix α2, wedging between the two motifs. The conserved C53 and C56 residues reside in the C-terminal part of strand β2 and the first turn of helix α2, respectively, and complete the zinc coordination sphere. The fold of ZAD_{Grau} appears to be critically dependent on the zinc coordination. This aspect of the structure (see Figures 3A-3C) is consistent with the results obtained with EDTA-dependent precipitation of ZAD_{Grau}. The two pairs of coordinating cysteines are approximately 50 residues apart. Zinc-coordination, therefore, links the β2-α2 transition region at the center of the molecule with the N-terminus of the domain likely to solidify the structure. In this view, the position of the zinc ion allows the fold to constrain the slanting angle of the C-terminal helix, α2, with respect to the long axis of the β-sheet (see Figures 3B-3C). The relative orientation of this long helix and the β-sheet is further defined by helix α1, which rests with one of its surfaces on the end of the β-sheet distal to the zinc ion and the adjacent loop regions. With a neighboring surface, helix α1 is in contact with the N-terminal and central portions of helix α2. Both the β1-sheet and the α1-α2 associations are based on extensive hydrophobic contacts forming a considerable hydrophobic core (α1: V29, V33, L34, H37, F38; β1/β2: L18, I20, I52; α2: W57, V60, F63, H64, Y67; Figures 1A, 3A-3C). In addition, the conserved H37 residue (atom NE2) of helix α1 hydrogen bonds at the edge of this core to the conserved H64 (atom ND1) of helix α2. In conclusion, the L2/α1/L3 region between the two central β-strands and the zinc ion provide platforms which orient the C-terminal helix relative to the globular portion of the ZAD_{Grau} molecule.

To further support the overall structure of ZAD_{Grau} obtained by X-ray crystallography, the inventors took circular dichroism spectra of the molecule in solution (data not shown). For far UV-CD measurements, the sample was dialyzed against 10mM Na₂HPO₄, pH 7.9. The spectra (190-260nm) were recorded with a JASCO J-720 spectropolarimeter (Jasco Corporation, Tokyo, Japan) at 20°C in a 0.1cm cuvette with a scanning speed of 50nm/min. 30 scans were averaged and baseline corrected. The spectra were analyzed using the neural network-based CD deconvolution software CDNN v2.1 (Böhm et al., 1992).

The calculated secondary structure content (45-50% helix, 5-10% strands) obtained from these recordings compare favorably to those seen in the crystal structure (37% helix, 6% strands). The conservation of length, predicted secondary structure, arrangement of the annotated secondary structure elements and the critical amino acid residues of ZADs (Chung et al., 2002; Figure 1A) suggest that the present ZAD_{Grau} crystal structure provides a prototype for ZAD folding.

### Crystal Structure Suggests ZAD_{Grau} Homodimers

The evolutionary restriction of ZADs to certain C₂H₂ zinc finger genes in insects, the chromosomal clustering of the majority of these ZAD-containing genes and the unique folding characteristics of the domain next to the DNA-binding domain of transcriptional regulators such as Grauzone strongly argue that the ZAD is associated with a specific and distinct biological function. However, the mere similarity to the fold group of the TCZFs does not allow any conclusions about the specific role of ZAD, because treble-clef motifs embody functions as diverse as binding to nucleic acids, proteins as well as small ligands and some may even exert enzymatic activity (Grishin, 2001).

However, the crystal structure of ZAD_{Grau} clearly supports a model, in which ZAD represents a protein-protein interaction module involved in homodimerization or interaction with other proteins. Figures 4A and 4B show that in the crystal two ZAD_{Grau} molecules are associated through a twofold axis in an isologous head-to-tail fashion. Figure 4A depicts a stereo ribbon plot of a ZAD_{Grau} dimer as seen in the crystal. The two subunits are denoted Mol I and Mol II and the two zinc centers are depicted in ball-and-stick. N- and C-termini are labeled. The orientation of the rear subunit is the same as in Figure 3B. Figure 4B shows a stereo representation of residue interactions in the dimer interface, 90° from the view in Figure 4A. The two ZAD_{Grau} subunits are rendered semitransparent to clearly reveal the interacting residues in ball-and-stick. All residues and the molecular termini are labeled.

As revealed by the protein-protein interaction server (http://www.biochem.ucl.ac.uk/ bsm/PP/server/), the dimer contact buries ∼1000Å² of accessible surface area (ΔASA) . This value seems on the lower side when compared to known homodimers (Jones and Thornton, 1996). However, the interaction surface of ZAD_{Grau} covers a total of close to 20% of the entire surface area. More importantly, a large number of amino acid residues, which are strongly conserved among ZAD family members (for details see Chung et al., 2002), are responsible for the contacts between the two subunits (see Figure 4B). When the conserved residues are mapped on the surface of ZAD_{Grau}, it becomes obvious that the largest conserved surface patch closely coincides with the presumed dimer interface. In particular, hydrophobic residues of the long C-terminal helix α2 (F63, F66, I70, Y77; Figure 1A) build up major parts of the contact interface (Figure 4B). As a consequence, the dimer interface is composed of 72.5% of the non-polar amino acid residues. The presumed dimer interface is thus designed very differently from the remainder of the surface, which is lined with polar residues and exhibits a highly negative electrostatic potential. In the region where the tip of helix α2 from one molecule contacts the globular portion of the other subunit, some intermolecular hydrogen bonds are observed as well (Q74-R5; Y77-E47; Figure 4B).

Because of the involvement of its hydrophobic side chains, the presumed homodimerization mode provides a straightforward explanation for the amphipathic design and the unusual length of helix α2 (Figure 3A). Since, in addition, because both helix α2 and the globular portion of ZAD are involved in this dimerization mode, the importance of restraining their relative orientations by the inserted L2/α2/L3 module and the zinc coordination becomes obvious. Further supporting its significance, the present dimerization may explain the lethal phenotype observed with the conserved R4 (corresponding to R5 in Grauzone) mutated to glycine in Zw5 (Gaszner et al., 1999). Its side chain is positioned by ionic interactions with D46 to engage in a hydrogen bond with Q74 of the neighboring molecule (Figure 4). In any case, the analysis of the crystal packing strongly suggests a functional homodimerization of ZAD_{Grau} ·
Besides the above symmetrical contact between two ZAD_{Grau} molecules, other crystal contacts were observed. Some of these are not functional because of an unreasonably small ΔASA. Other contacts did not involve conserved residues. One rather intimate alternative association takes place through the crystallographic fourfold screw axis. However, this symmetry element gives rise to heterologous contacts, leaving the bonding potentials of ZAD unsaturated. The latter interaction mode could therefore lead to the formation of larger oligomers. The inventors therefore examined the arrangement of ZAD in solution.

### Example 4: Examination of the dimerization of ZAD_{GRAU} in solution by Chemical cross-linking and Multi-angle-laser-light scattering:

The quaternary structure of ZAD_{Grau} in solution was investigated by two approaches. First, the inventors performed chemical crosslinking experiments with glutaraldehyde (see Figure 5A). The crosslinking reactions were carried out at 25°C in 20mM Tris-HCl, pH 7.5, 100mM NaCl with 15µM samples of ZAD_{Grau}, GST (26kDa), and SNAP-25 (25kDa) (Fasshauer et al., 1999) and 1mM glutaraldehyde. The reactions were stopped at selected time points by boiling in SDS sample buffer.

In contrast to SNAP-25, which was reported not to self-interact (Fasshauer et al., 1999), and in parallel with the dimerizing GST, ZAD_{Grau} could be efficiently crosslinked to the dimer state, but no higher oligomers were observed. Figure 5A shows the results from chemical crosslinking with glutaraldehyde displayed on a denaturing polyacrylamide gel. Crosslinking took place for the times indicated above the lanes. SNAP-25 served as a negative control, GST as a positive control. Molecular weight markers are given on the left (kDa) .

Secondly, multi-angle-laser-light-scattering following size exclusion chromatography yielded strong evidence for homdimerization in solution (see Figure 5B). The experiment was performed on a HR-10/30 Superdex-200 size exclusion column (Amersham) connected to a UV spectrometer and the Dawn and Optilab instruments XY (Wyatt Technology Corp.). 200µl of a 25µM ZAD_{Grau} sample were chromatographed in 20mM Tris-HCl, pH 8.0, 150mM NaCl. The UV₂₈₀ absorption, the light scattering at 632.8nm and the differential refraction of the elution profile were monitored. Spectra were analyzed with the Astra software package (Wyatt, 1993). The inventors also checked ZAD_{Grau} migration on an analytical Superdex-75 column in comparison to protein molecular weight standards.

Figure 5B shows the results from a gel filtration chromatography run combined with multi-angle-laser-light scattering analysis of the emerging peak. Emergence of protein from the column was detected by absorbance at 280nm (black trace, left y-axis), readout from the light scattering of the single, symmetrical peak is depicted as a gray line 10 (right y-axis).

ZAD_{Grau} eluted as a single symmetrical peak from various size exclusion columns with different optimal separation regimes. Comparison of the elution times with those of reference proteins was consistent with ZAD_{Grau} dimers (not shown) . The scattering signal at 632.8nm across the elution peak indicated a molecular weight of 21.11kDa, which matched the theoretical dimer mass (21.14kDa) almost perfectly (Figure 5B). This high congruence and the lack of a monomer or a trimer signal, strongly suggested a monodisperse dimer solution. Collectively, our results provide strong evidence for ZAD_{Grau} homodimerization under near-physiological salt conditions. They leave the twofold symmetrical association of ZAD_{Grau} in the crystal (see Figures 4A and 4B) as the only dimerization mode, which is consistent with all observations. Because ZAD_{Grau} is an independently folding unit, dimerization is expected to prevail within the context of the full-length Grauzone transcription factor. By extension of this finding, a general function of ZADs could be to provide dimerization modules that mediate homodimer and/or heterodimer formation among closely related members of the ZAD-transcription factor family.

In support of this observation, homodimerization was also reported for the ZAD-containing transcription factor Sry-δ (Payre et al., 1997; Ruez et al.,1998). It is noteworthy, that Sry-δ comprises a deviated version of ZAD family members and could thus not be considered as prototype for ZAD function (Chung et al., 2002).

### ZAD_{Grau} Lacks DNA-Binding Features

The majority of zinc finger proteins were shown to be nucleic acid binding proteins (Berg and Shi, 1996; Laity et al., 2001; Pavletich and Pabo, 1991; Wolfe et al., 2000). Because of this amply documented function, the inventors asked whether the ZAD_{Grau} structure has features to support nucleic acid binding as well. In order to obtain first hints, the inventors compared the ZAD with the structure of the C-terminal treble-clef module of the chicken erythroid transcription factor GATA-1 (Tjandra et al., 1997), a representative of sequence-specific DNA-binding TCZFs (treble-clef zinc finger). The two molecules display opposite electrostatic surface potentials. Whereas the TCZF of GATA-1 exhibits an almost continuously electropositive surface potential that facilitates a close association with the negatively-charge sugar-phosphate backbone of the DNA, the ZAD_{Grau} is almost completely wrapped into negative potential (not shown). This observation strongly argues against DNA binding features of ZAD.
Superimposition of one subunit of a ZAD_{Grau} dimer on the structure of DNA-bound GATA-1 TCZF (Tjandra et al., 1997) revealed that the second ZAD_{Grau} module faces the minor groove of the DNA and would thus collide with the DNA backbone (not shown). Furthermore, Grauzone mutants, which lack the ZAD-containing region of the protein, had unaltered DNA-binding properties *in vitro,* whereas the part of Grauzone protein that contains the ZAD in the absence of C₂H₂ zinc fingers had no DNA-binding activity (Chen et al., 2000), supporting the argument that ZAD_{Grau} has no DNA-binding features. Since the key positions and features of the amino acid residues are conserved among ZADs (for an alignment see Chung et al., 2002), it appears unlikely that any of them carries DNA-binding properties. The inventors therefore propose that ZADs, in general, are protein interaction modules involved in homodimerization and/or the formation of protein complexes.

To further support this conclusion, the inventors also compared the ZAD_{Grau} dimer with the DNA-bound dimer of other members of the TCZF fold group, the glucocorticoid receptors (Luisi et al., 1991; Grishin, 2001). In these receptor molecules, the DNA binding helix equivalent to ZAD helix α2 is employed as a recognition element, which is positioned in the DNA major groove. Two DNA binding domains interact through their C-terminal helical extension, which have no equivalent in ZADs. These C-terminal extensions yield a spacing between the two recognition helices from neighboring subunits, which is large enough to position them both into neighboring turns of the DNA major groove. Therefore, the dimerization mode of ZADs is fundamentally different from the interaction mode seen in the DNA binding domains of glucocorticoid receptors, consistent with these molecules serving different functions.

### Example 5: Identification of a putative Grauzone ZAD homologue in the Anopheles gambiae genome:

The inventors searched the whole Anopheles *gambiae* genome using the ZAD HMM described in Chung et al. 2002 and the Wise package 2.2.0 (Birney et al., 1996). All identified ZADs were aligned with all *Drosophila melanogaster* ZADs using ClustalW 1.8.1. (Thompson et al., 1994), false positive hits were eliminated. The alignment was used to construct a neighbor-joining tree with ClustalW. Based on this tree the inventors identified the closest relative of the Grauzone ZAD in the *Anopheles gambiae* genome. The alignment of the Grauzone ZAD with the closest paralog, CG15073, of *Drosophila melanogaster* and the putative Anopheles homologue was performed with ClustalW using default parameters (see Figure 1A).

Figure 1B shows a primary sequence alignment of ZAD_{Grau} denoted as Grauzone with homologous protein domains from *Droso phila melanogaster* or *Anopheles gambiae*. The primary sequences of ZAD_{Grau} from *D.melanogaster* and the putative ZAD from *A.gambiae* (denoted as Grauzone *A.gambiae*) exhibit a high degree of primary sequence homology of around 50% and a primary sequence identity of around 38% as determined with the program EMBOSS-Align (Rice,P. Longden, I. and Bleasby,A. (2000) "EMBOSS: The European Molecular Biology Open Software Suite" Trends in Genetics 6(6.):276-277). The alignment was done with the program ClustalW 1.81 (Thompson et al., 1994). The four cysteines coordinating the zinc are shown with a grey background. Similar residues are shown in grey letters.

Figure 6 shows the structural coordinates of ZAD_{Grau} as determined by the above-described x-ray structure analysis (see example 3). The structure coordinates refer to mathematical coordinates derived from mathematical equations related to the patterns obtained on diffraction of a monochromatic beam of x-rays by the atoms of the crystal of ZAD_{Grau}. The diffraction data are used to calculate an electron density map of the asymmetric unit of the crystal. The electron density map shows the electron distribution within the crystal and can be used to build a 3-dimensional model of ZAD_{Grau}. It is common knowledge of experts in the field of protein x-ray crystallography, that structurally similar proteins exhibiting the same overall 3-dimensional fold have a root mean square deviation from the backbone atoms of the coordinates of ZAD_{Grau} shown in this Figure 6 of not more than 1.5 Å. In Figure 6 the structural coordinates of ZAD_{Grau} are given as a table of structural coordinates in protein data bank (PDB) format, including the values X, Y, Z and B for each of the atoms in the amino-acids in ZAD_{Grau}. The term "atom type" refers to the type of atom of the respective amino acid, for example the nitrogen-atom of the amide backbone (N), C-α (CA), C-β (CB), C-γ (CG), C-δ (CD) atoms or oxygen atoms. The term "residue" refers to the type of amino-acid given in the three letter code, to which the aforementioned atom belongs, for example aspartate (ASP) or isoleucine (ILE). The technical terms "X, Y, Z" refer to the crystallographically defined atomic position of the atoms calculated with respect to a specially defined crystallographic origin. The term "B" refers to a thermal factor, that measures the mean displacement of the atom around its atomic center in crystals of ZAD_{Grau}.

The scope of protection of the invention is not limited to the examples given hereinabove. The invention is embodied in each novel characteristic and each combination of characteristics, which includes every combination of any features which are stated in the claims, even if this combination of features is not explicitly stated in the claims.

### References

Berg, J. and Shi, Y. (1996). The Galvanization of Biology: A Growing Appreciation for the Roles of Zinc. *Science 271,* 1081-85.
Bhaskar, V., Valentine, S. and Courey, A. (2000). A functional interaction between dorsal and components of the smt3 conjugation machinery. *J Biol Chem 275*, 4033-40.
Birney, E., Thompson, J., and Gibson, T. J. (1996). PairWise and SearchWise: finding the optimal alignment in a simultaneous comparison of a protein profile against all DNA translation frames. *Nucleic Acids Res 24*, 2730-39.
Blanton, J., Gaszner, M. and Schedl, P. (2003). Protein:protein interactions and the pairing of boundary elements in vivo. *Genes Dev 17*, 664-75.
Böhm, G., Muhr, R. and Jaenicke, R. (1992). Quantitative analysis of protein far UV circular dichroism spectra by neural networks. *Protein Eng 5*, 191-5.
Brunger, A., Adams, P., Clore, G., DeLano, W., Gros, P., Grosse-Kunstleve, R., Jiang, J., Kuszewski, J., Nilges, M., Pannu, N., Read, R., Rice, L., Simonson, T. and Warren, G. (1998). Crystallography & NMR system: A new software suite for macromolecular structure determination. *Act Cryst D 54,* 905-921.
Chen, B., Harms, E., Chu, T., Henrion, G. and Strickland, S. (2000). Completion of meiosis in Drosophila oocytes requires transcriptional control by grauzone, a new zinc finger protein. Development. 127, 1243-51.
Chu, T., Henrion, G., Haegeli, V. and Strickland, S. (2001). Cortex, a Drosophila gene required to complete oocyte meiosis, is a member of the Cdc20/fizzy protein family. Genesis 29, 141-52.
Chung, H., Schäfer, U., Jäckle, H. and Böhm, S. (2002). Genomic expansion and clustering of ZAD-containing C₂H₂ zinc-finger genes in Drosophila. EMBO Rep. 3, 1158-62.
Collaborative Computational Project 4. (1994). The CCP4 Suite: Programs for protein crystallography. Acta Cryst D 50, 760-63.
Collins, T., Stone, J. and Williams, A. (2001). All in the family: the BTB/POZ, KRAB, and SCAN domains. Mol Cell Biol. 21, 3609-15.
Crozatier, M., Kongsuwan, K., Ferrer, P., Merriam, J., Lengyel, J., and Vincent, A. (1992). Single amino acid exchanges in separate domains of the Drosophila serendipity delta zinc finger protein cause embryonic and sex biased lethality. Genetics 131, 905-16.
Daquber-Osguthorpe,P., Roberts, V.A., Osguthorpe, D.J., Wolff, J., Genest, M. and Hagler, A.T. (1988). Structure and energetics of ligand binding to proteins: *Escherichia coli* dihydrofolate reductase-trimethoprim, a drug-receptor system. Proteins:Struc. Funct. Genet., 4, 31-47
Fasshauer, D., Antonin, W., Margittai, M., Pabst, S. and Jahn, R. (1999). Mixed and non-cognate SNARE complexes. Characterization of assembly and biophysical properties. J Biol Chem 274, 1440-6.
Gaszner, M., Vazquez, J. and Schedl, P. (1999). The Zw5 protein, a component of the scs chromatin domain boundary, is able to block enhancer-promoter interaction. Genes Dev. 13, 2098-107.
Grishin, N. (2001). Treble clef finger - a functionally diverse zinc-binding structural motif. Nucl acid res 29, 1703-14.
Guex, N. and Peitsch, M. (1997). SWISS-MODEL and the Swiss-PdbViewer: an environment for comparative protein modeling. Electrophoresis 18, 2714-23.
Harms, E., Chu, T., Henrion, G. and Strickland, S. (2000). The only function of Grauzone required for Drosophila oocyte meiosis is transcriptional activation of the cortex gene. Genetics. 155, 1831-9.
Jones, S. and Thornton, J. (1996). Principles of protein-protein interaction. PNAS 93, 13-20.
Klug, A. and Schwabe, J. (1995). Protein motifs 5: Zinc fingers. FASEB J 9, 597-604.
Kraulis, P. (1991). MOLSCRIPT: a program to produce both detailed and schematic plots of protein structures. J Appl Cryst 24, 946-50.
Krishna, S., Indraneel, M. and Grishin, N. (2003). Structural classification of zinc fingers: SURVEY AND SUMMARY. Nucl. Acids. Res. 31, 532-50.
Laity, J., Lee, M., and Wright, P. (2001). Zinc finger proteins: new insights into structural and functional diversity. Curr Op Struct Biol 11, 39-46.
Lander, ES. et al. (2001). Initial sequencing and analysis of the human genome. Nature 409, 860-921.
Laskowski, R., MacArthur, M., Moss, D. and Thornton, J. (1993). PROCHECK: a program to check the stereochemical quality of protein structures. J Appl Cryst 26, 283-291.
Lespinet, O., Wolf, YI.. Koonin, EV. and Aravind, L. (2002). The role of lineage-specific gene family expansion in the evolution of eukaryotes. Genome Res. 12, 1048-59.
Looman, C., Abrink, M., Mark, C. and Hellman, L. (2002). KRAB Zinc Finger Proteins: An Analysis of the Molecular Mechanisms Governing Their Increase in Numbers and Complexity During Evolution. Mol Biol Evol. *19*, 2118-30.
Lorick, K., Jensen, J., Fang, S., Ong, A., Hatakeyama, S. and Weissman, A. (1999). RING fingers mediate ubiquitin-conjugating enzyme (E2)-dependent ubiquitination. PNAS 96, 11364-11369.
Luisi, B., Xu, W., Otwinowski, Z., Freedman, L., Yamamoto, K. and Sigler, P. (1991). Crystallographic analysis of the interaction of the glucocorticoid receptor with DNA. Nature 352, 497-505.
Luzzati, V. (1952). Traitement statistique des erreurs dans la determination des structures cristallines. Acta Cryst A 5, 802-10.
Mackay, J. and Crossley, M. (1998). Zinc fingers are sticking together. Trends Biochem Sci 23, 1-4.
Margolin, J., Friedman, J., Meyer, W., Vissing, H., Thiesen, H. and Rauscher, F. (1994). Kruppel-associated boxes are potent transcriptional repression domains. PNAS 91, 4509-13.
McCarty, A., Kleiger, G., Eisenberg, D. and Smale, T. (2003). Selective dimerization of a C₂H₂ Zinc Finger Subfamily. Mol Cell 11, 459-470.
Merritt, E., and Bacon, D. J. (1997). Raster3D:photorealistic molecular graphics. Meth Enzymology *277*, 505-524.
Miller, J., McLachlan, A. and Klug, A. (1985). Repetitive zinc-binding domains in the protein transcription factor IIIA from Xenopus oocytes. EMBO J 4, 1609-14.
Otwinowski, Z. and Minor, W. (1997). Processing of X-ray diffraction data collected in oscillation mode. Methods Enzymol. *276*, 307-326.
Pavletich, N. and Pabo, C. (1991). Zinc finger-DNA recognition: crystal structure of a Zif268-DNA complex at 2.1 A. Science *252*, 809-17.
Payre, F., Buono, P., Vanzo, N., and Vincent, A. (1997). Two types of zinc fingers are required for dimerization of the serendipity delta transcriptional activator. Mol Cell Biol. *17*, 3137-45.
Payre, F., Crozatier, M. and Vincent, A. (1994). Direct control of transcription of the Drosophila morphogen bicoid by the serendipity delta zinc finger protein, as revealed by in vivo analysis of a finger swap. Gens Dev. *8*, 2718-28.
Peng, H., Begg, G., Harper, S., Friedman, J., Speicher, D., and Rauscher, F. (2000). Biochemical analysis of the Kruppel-associated box (KRAB) transcriptional repression domain. J Biol Chem. *275*, 18000-10.
Rosenberg, H., Schröder, C., Preiss, A., Kienlin, A., Cote, S., Riede, I. and Jäckle, H. (1986). Structural homology of the product of the Drosophila Krüppel Gene with Xenopus transcription factor-IIIA. Nature *319*, 336-339.
Ruez, C., Payre, F., and Vincent, A. (1998). Transcriptional control of Drosophila bicoid by Serendipity delta: cooperative binding sites, promoter context, and co-evolution. Mech Dev. *78*, 125-34.
Thompson, J., Higgins, D., and Gibson, T. (1994). CLUSTAL W: improving the sensitivity of progressive multiple sequence alignment through sequence weighting, position-specific gap penalties and weight matrix choice. *Nucleic Acids Res 22,* 4673-80.
Tjandra, N., Omichinski, J., Gronenborn, A., Clore, G. and Bax, A. (1997). Use of dipolar 1H-15N and 1H-13C couplings in the structure determination of magnetically oriented macromolecules in solution. Nat Struct Biol *4*, 732-38.
Turk, D. (1996). MAIN 96: An interactive software for density modifications, model building, structure refinement and analysis. In Bourne, P.E. and Watenpaugh, K. (eds.), Meeting of the International Union of Crystallography Macromolecular Macromolecular Computing School. International Union of Crystallography.
Weill, M., Lutfalla; G., Mogensen, K., Chandre, F., Berthomieu, A., Berticat, C., Pasteuer, N., Philips, A., Fort, P. and Raymand, M. (2003), Insecticide resistance in mosquito vectors. Nature, 423, 136-137.
Wolfe, S., Nekludova, L. and Pabo, C. (2000). DNA recognition by Cys2His2 zinc finger proteins. Annu Rev Biophys Biomol Struct *29*, 183-212.
Wyatt, P. (1993). Light scattering and the absolute characterisation of macromolecules. Anal Chim Acta *272*, 1-40.

## Claims

1. Method for obtaining an agent, which interacts with a ZAD_{Grau} molecule or a molecule with similar fold to ZAD_{Grau}, comprising the following steps:
a) using the structural coordinates of
i) at least a portion of the structural coordinates of the ZAD_{Grau} amino acids shown in Fig. 6, or
ii) at least a portion of a structurally similar molecule of ZAD_{Grau}, having a root mean square deviation from the backbone of the said ZAD_{Grau} amino acids of not more than 1.5 Å,
to generate a three-dimensional model of the portion of ZAD_{Grau} or the molecule with similar fold to ZAD_{Grau},
b) employing the three-dimensional model and at least one molecular modeling technique to select or design an interacting agent.

2. Method according to claim 1,
- wherein in step a) the structural coordinates of
i) at least two of the following ZAD_{Grau} amino acids shown in Fig. 6:
- R5, L6, E47, Q59, I70, Q74, Y77, H37-V42 and F63-V68, or
ii) a portion of a structurally similar molecule of ZAD_{Grau}, having a root mean square deviation from the backbone atoms of said amino acids of not more than 1.5 Å
is used.

3. Method according to one of the claims 1 or 2, further comprising the following steps:
c) synthesising said interacting agent and
d) bringing said agent in contact with the binding site of the molecule or structural similar molecule to detect the interaction.

4. Method according to one of the claims 1 to 3,
- wherein step b) comprises:
- screening a library of individual chemical entities for the agent by performing a computer assisted docking operation between the individual chemical entities and the portion of the three-dimensional model.

5. Method according to one of the claims 1 to 3,
- wherein step b) comprises:
- designing of the agent by assembling individual chemical entities, binding to the portion of the three-dimensional model into a single interacting agent and docking this single interacting agent to the portion of the three-dimensional model using computational means.

6. Method according to one of the claims 4 or 5,
- wherein step b) further comprises:
- analysing the results of the docking operation to evaluate the interaction between the agent and the portion of the three-dimensional model of the molecule or structural similar molecule.

7. Method according to one of the claims 4 to 6,
- wherein step b) further comprises:
- performing an energy minimisation or molecular dynamics operation using computational means to improve the interaction between the agent and the portion of the three-dimensional model.

8. Method according to claim 3,
- wherein step d) comprises orally administering the interacting agent to flies and detecting the rate of dying or infertile flies.

9. Method according to one of the claims 1 to 8,
- wherein in step b) the interacting agent is selected or designed from a molecule comprising an overall structure similar to ZAD_{Grau}.

10. Use of an agent obtaining by a method according to one of the claims 1 to 9 as an insecticide.

11. A Method of obtaining a three-dimensional model of an ZAD_{Grau}-like molecule of unknown structure, comprising the following step:
A1) using
- computational means,
- structural and/or sequence information of the ZAD_{Grau}-like molecule of unknown structure and
- at least a portion of the structural coordinates of the ZAD_{Grau} molecule shown in Fig. 6
to obtain the three-dimensional model of the ZAD_{Grau}-like molecule.

12. Method according to the previous claim,
- wherein step A1) comprises the following steps:
A11) obtaining x-ray diffraction data of a crystallized ZAD_{Grau}-like molecule,
A12) using the portion of the structural coordinates of the ZAD_{Grau} molecule to obtain an electron density map of the ZAD_{Grau}-like molecule, and
A13) building of a three-dimensional model of the ZAD_{Grau}-like molecule into the electron density map.

13. Method according to claim 11,
- wherein step A1) comprises:
using a homology modeling program and the primary sequence of the ZAD_{Grau}-like molecule to obtain the three-dimensional model of the ZAD_{Grau}-like molecule.

14. Method according to one of the claims 11 to 13,
- wherein step A1) comprises:
- generating a primary sequence alignment between ZAD_{Grau}-and the ZAD_{Grau}-like molecule and adjusting a three-dimensional model of ZAD_{Grau} according to the primary sequence alignment and using this model as a portion of the structural coordinates of the ZAD molecule.

15. Method according to one of the previous claims 11 to 14,
- wherein the ZAD_{Grau}-like molecule is selected from the following molecules:
- ZAD-like molecules from *A. gambiae*, *D. melanogaster.*

16. A crystal comprising ZAD molecules.

17. The crystal according to the previous claim,
- further diffracting X-rays to a resolution of at least 3 Å.

18. The crystal according to claim 17,
- wherein the ZAD molecules are ZAD_{Grau.}

19. The crystal according to claim 17,
- wherein the ZAD molecules comprise
i) ZAD_{Grau} molecules with the structural coordinates shown in Fig. 6, or
ii) structurally similar molecules of ZAD_{Grau}, comprising structural coordinates with a root mean square deviation from the backbone atoms of the coordinates shown in Fig. 6 of not more than 1.5 Å.

20. A computer-system for generation and computational utilization of a three-dimensional model of ZAD, comprising the following components:
I) a computer readable medium having stored thereon
i) at least a portion of the structural coordinates of all the ZAD_{Grau} amino acids shown in Fig. 6, or
ii) at least a portion of a structurally similar molecule of ZAD_{Grau}, having a root mean square deviation from the backbone of the said ZAD_{Grau} amino acids of not more than 1.5 Å, or
iii) x-ray diffraction data of ZAD_{Grau} or a structurally similar molecule of ZAD_{Grau},
II) computing subroutines for the generation and computational utilization of the said structural coordinates,
III) a central processing unit for performing said computing subroutines.

21. The computer-system according to the previous claim,
- wherein the computing subroutines of component II) are selected from the group consisting of
- x-ray data processing, reduction, indexing, merging, intensity scaling, truncation,
- molecular replacement, molecular modeling.
